# EUROPEAN PATENT APPLICATION

(11) **EP 4 840 450 A1**
(43) Date of publication of application: **18.11.2026**
(21) Application number: 25175644.1
(22) Date of filing: 12.05.2025
(51) Int. Cl.: A61K 31/426, A61K 31/428, A61P 35/00, A61P 35/04

(54) **HETEROCYCLIC INHIBITORS OF S100A4 TRANSCRIPTION FOR USE IN THE TREATMENT OF CANCER AND METASTASES**

(71) Applicant: Charité - Universitätsmedizin Berlin Körperschaft des öffentlichen Rechts, 10117 Berlin (DE); Max-Delbrück-Centrum für Molekulare Medizin in der Helmholtz-Gemeinschaft, 13125 Berlin (DE)
(72) Inventor: STEIN, Ulrike, 16341 Panketal (DE); SCHÖPE, Paul, 13156 Berlin (DE); KOBELT, Dennis, 16552 Schildow (DE); HEISTERKAMP, Nina, 12163 Berlin (DE); SCHÜTZ, Devin, 12621 Berlin (DE)
(74) Representative: Hertin und Partner Rechts- und Patentanwälte PartG mbB

(57) **Abstract**

The invention relates to chemical compounds useful as inhibitors of S100A4 in the treatment of cancer. The invention further relates to the medical use of the chemical compounds in the treatment of a medical condition associated with S100A4 transcription. The invention further relates to pharmaceutical compositions comprising a compound of the present invention or a pharmaceutically acceptable salt thereof and a pharmaceutically acceptable carrier.

## Description

The invention is in the field of biochemistry, pharmaceutical compounds and medical uses thereof.

The invention relates to chemical compounds and their medical use as transcriptional inhibitors of S100A4. The invention further relates to a pharmaceutical composition comprising the chemical compounds of the invention, and use of the compounds or compositions in the treatment of a tumor disease, such as a solid tumor, and/or for the treatment and/or prophylaxis of tumor metastasis.

### BACKGROUND OF THE INVENTION

Colorectal cancer (CRC) represents an unmet clinical need. CRC is the third most common cancer globally, and further, the second-leading cause of cancer related deaths worldwide [1]. It is well-defined that metastasis is the most lethal attribute of CRC. Despite the implementation of early detection programs, about 20-25% of all patients have already synchronous metastases at the time of diagnosis and approximately 25-50% will develop metastases in the course of disease, mainly in liver and lung [30]. The cancer cell dissemination process reduces patient survival by accounting for 80.2% of all CRC deaths [2]. Furthermore, those diagnosed in not distantly metastatic stages I-III have a 5-year survival rate (YSR) of 82.4%, whereas metastatic stage IV patients exhibit a mere 15.7% [3]. Notably, not even patients in stages I-III avoid this deadly spread: approximately 20% of patients initially diagnosed in stages I-III will experience distant metachronous metastasis normally within the first 3 years of their diagnoses [4]. This risk and prevalence of metastasis in CRC diagnoses emphasizes the clinical need to refine current treatment methods to target metastasis-associated molecular processes.

The S100 calcium-binding protein family member S100A4 represents a potent executer of CRC metastasis [5-9], and thus represents a target for therapeutic interference. S100A4 possesses a low molecular weight of 11.5 kDa [10] and is present at high levels in the blood and highly expressed in tumors of various entities including colorectal, ovarian, prostate, pancreatic, bladder, lung, esophageal, and gastric cancer [11-14]. It is considered an effective clinical prognostic biomarker, with high S100A4 expression levels being associated with reduced patient survival [15-17]. Furthermore, this metastasis-executing gene contains a Transcription Factor 4 (TCF4) binding site in its promoter region that results in it being a target of the canonical Wnt-signaling pathway through its binding with the heterodimeric β-catenin/TCF complex [9]. This signaling pathway is aberrant in > 90% of CRC cases, making S100A4 a pertinent target when treating CRC [18]. S100A4 fuels metastasis through a variety of mechanisms: firstly, S100A4 can bind extracellularly to RAGE [19] and TLR4 receptors [20], thus driving cell survival, hypoxia mitigation and chronic inflammation. Furthermore, S100A4 activity intracellularly includes binding to non-muscle myosin 2A, tropomyosin and F-actin to drive cellular motility [21-25], increasing matrix metalloproteinase production to degrade the ECM [26,27] and reducing cellular adhesion molecules such as E-cadherin [28,29].

These processes result in enhanced cellular motility through enabled epithelial-to-mesenchymal transition, thus heightening migration and metastasis capabilities. Therefore, restricting S100A4-mediated functions is a promising route to reduce metastasis formation in CRC patients.

The inventors previously identified the first transcriptional inhibitors of the metastasis gene S100A4 using a human S100A4 promoter based HTS [57-59]. Among the best candidates, FDA-approved nonsteroidal anti-inflammatory drug sulindac, the antibiotic calcimycin, and the anti-helminthic drug niclosamide (WO2012143377) were identified.

Despite the advancements in this field, there is still an unmet need to provide additional effective agents for the treatment of cancer, in particular tumor diseases, that inhibits tumor and metastasis progression, thereby optimizing treatment efficacy and improving clinical outcomes.

### SUMMARY OF THE INVENTION

In light of the prior art, one technical problem underlying the invention was to provide alternative or improved means for the treatment of cancer. Another technical problem may also be viewed as the provision of means for the treatment and/or reduction of risk of cancer progression, for example in early stage cancer patients. Another technical problem may also be viewed as the provision of alternative or improved means for treating and/or reducing the risk of metastasis in patients with solid tumors, for example in those with elevated S100A4 levels.

This problem is solved by the features of the independent claims. Preferred embodiments of the present invention are provided by the dependent claims.

In one embodiment, the invention relates to a compound according to **Formula 1** for use in the treatment of cancer 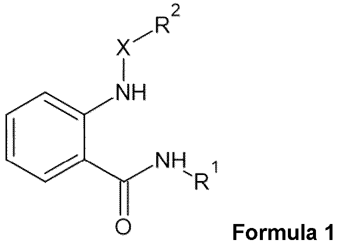 wherein
**X** is carbonyl -(C=O)- or sulfonyl -(SO₂)-,
**R¹** is a mono- or bicyclic heteroaryl group (preferably comprising N, S and/or O, more preferably an azole, thiazole or benzothiazol), optionally substituted with one or more of C1 to C5 alkyl (preferably C1 to C3 alkyl), halogen (preferably F, Cl and/or Br), nitro, alkyl sulfonyl (preferably C1 to C3), alkoxy (preferably OCH₂CH₃), haloalkyl (preferably comprising F), hydroxyl, acyl, aryl (preferably phenyl, optionally substituted with C1 to C5 alkyl (preferably C1 to C3 alkyl) or halogen (preferably F, Cl and/or Br), and
R² is a mono- or bicyclic aryl group (preferably phenyl) or a bicyclic structure comprising an aryl group (preferably benzodioxanyl), optionally substituted with one or more of C1 to C5 alkyl (preferably C1 to C3 alkyl), halogen (preferably F, Cl and/or Br), alkoxy (preferably OCH₂CH₃), haloalkyl (preferably comprising F, Cl and/or Br), hydroxyl, acyl.

The compounds described herein show activity as inhibitors of S100A4 transcription. The protein S100A4 is a member of the S100 family of proteins comprising 2 EF-hand calcium binding motifs. Proteins of this family are localized in the cytoplasm and/or nucleus of a wide range of cells, and involved in the regulation of a number of cellular processes such as cell cycle progression and differentiation. The protein S100A4 influences cell motility, cell invasion and tubulin polymerization, whereby chromosomal rearrangements and altered expression characteristics of this gene have been implicated in tumor metastasis.

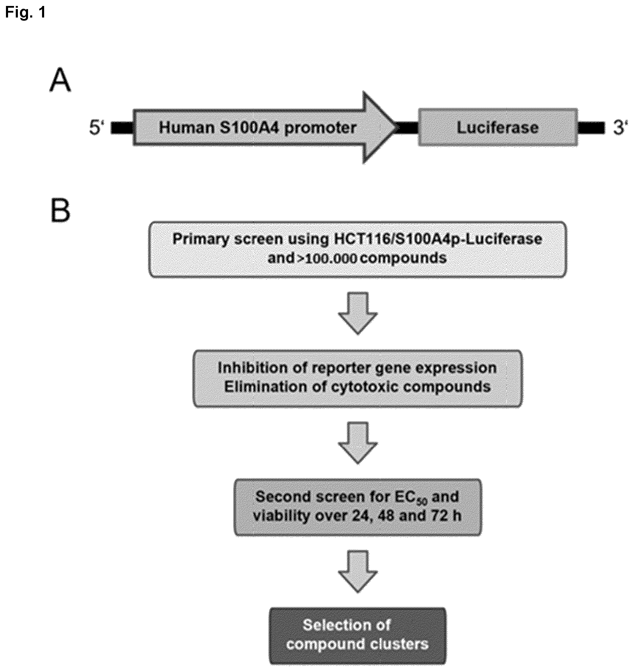

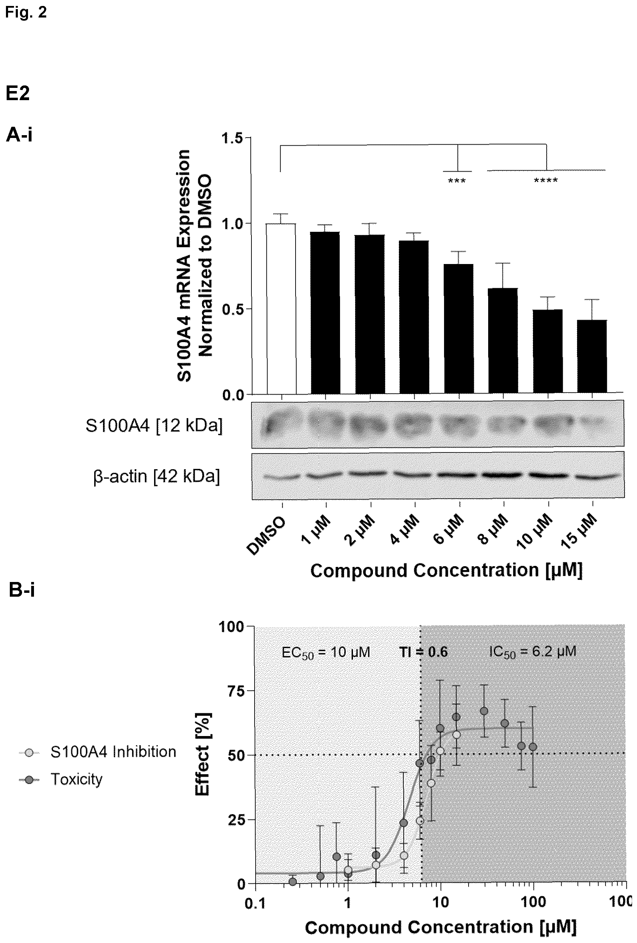

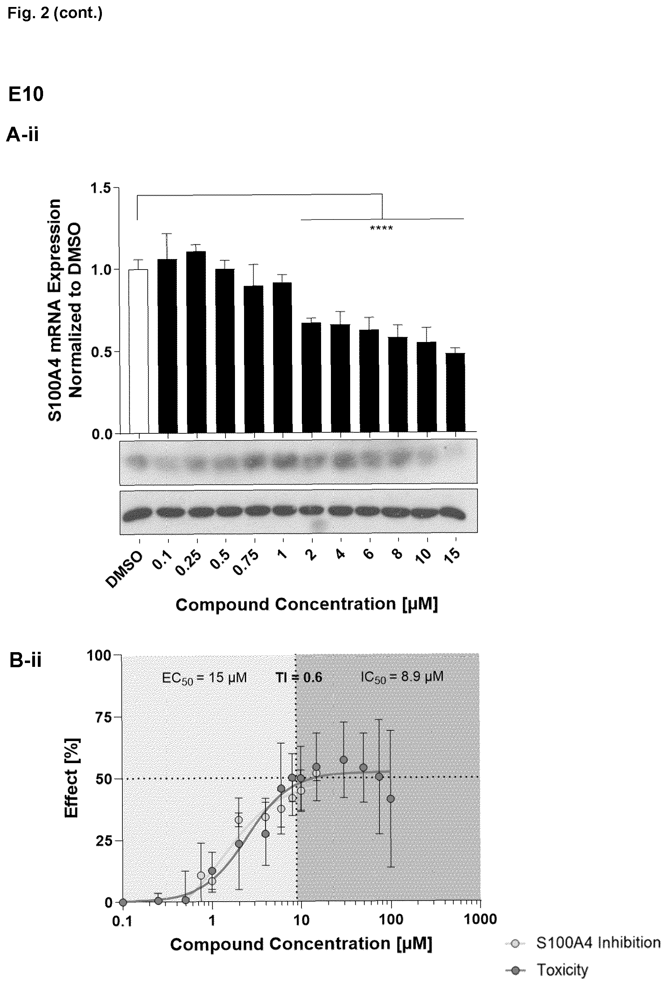

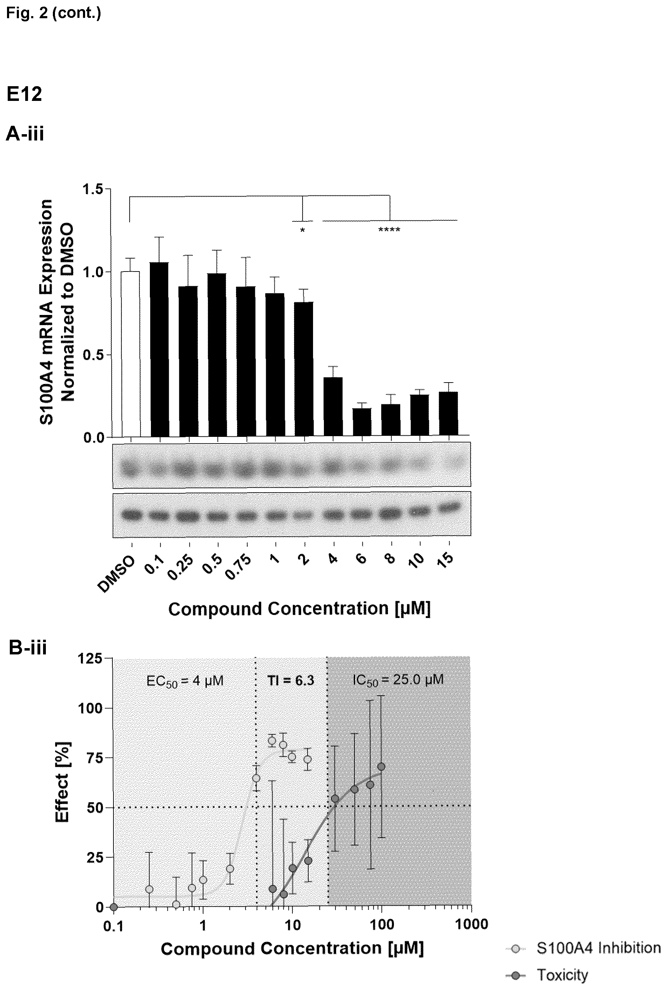

The compounds of the present invention were screened via a S100A4 promoter-driven luciferase construct transfected into HCT116 cells (See Figure 1). This procedure identified 3 clusters of compounds with varying characteristics, including the compounds designated herein as E2, E10 and E12. Consequently, these clusters were investigated relating to their transcriptional inhibition of S100A4, and their impact on migration and viability. Cluster E12, including the compound E12 and other compounds of the present invention, showed surprisingly favorable characteristics for use as a transcriptional inhibitor of S100A4 (See Figure 2).

The compounds of the present invention are useful as transcriptional inhibitors targeting S100A4 as a key driver of metastasis. The effect of inhibiting transcription of S100A4 leads to reduced cell motility and tumor progression and metastasis. Furthermore, this effect is evident both in vitro and in vivo experimental systems. The observed effects may translate into clinical settings, providing effective means in tumor treatment of mammalian, preferably human subjects. The compounds described herein exhibit surprisingly beneficial properties, such as the low EC50 values required for effective inhibition of the S100A4 transcription and the unexpectedly high Therapeutic Index of E12 (See Figure 2).

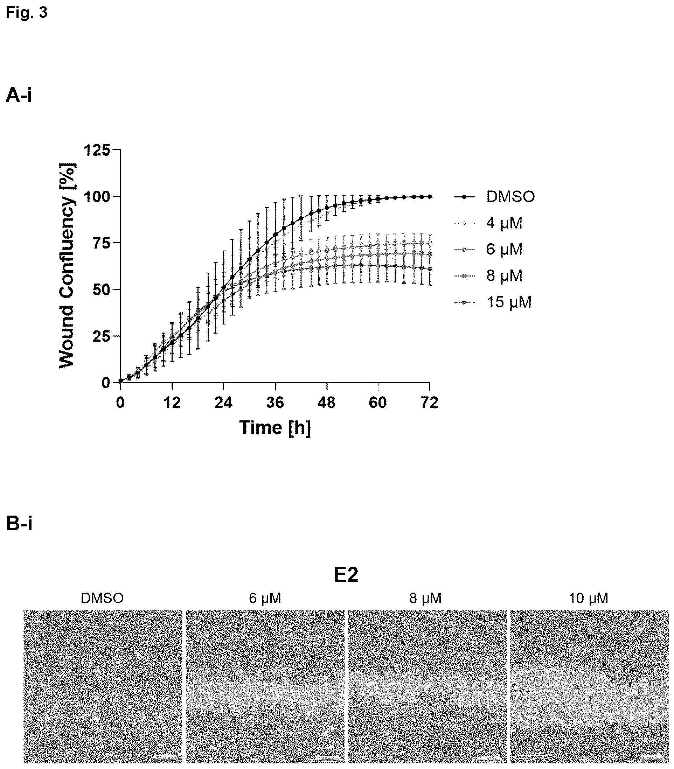

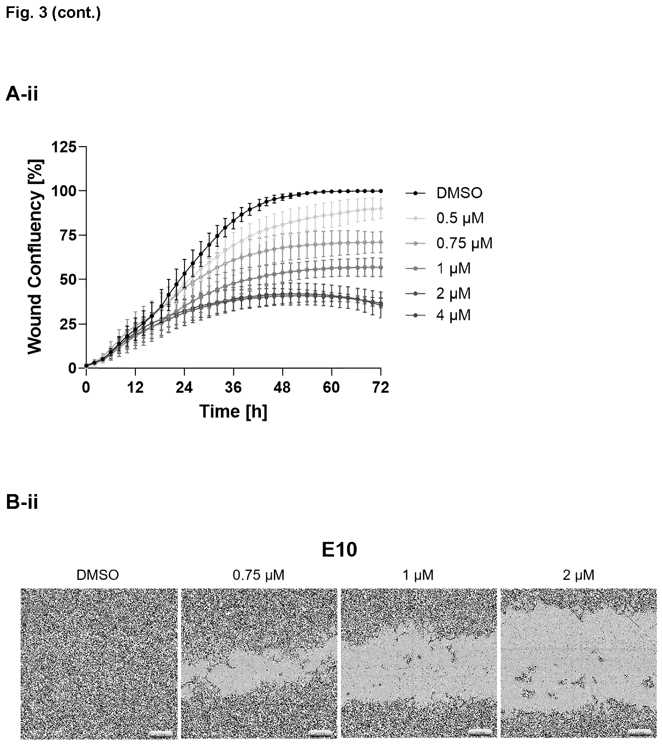

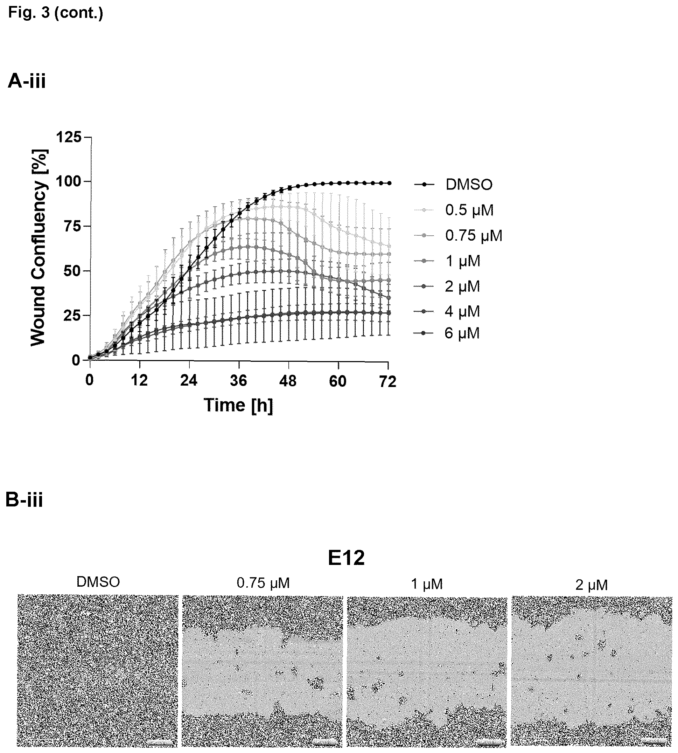

The observed concentration dependent inhibition is also evident for multiple compounds described by Formula 1. This supports the desired biological and therapeutic effects for the class of active agents as described herein without limitation to the particular agents tested. The inventors have established that by inhibiting the transcription of S100A4, beneficial effects in reduced cancer cell motility can be achieved. Further detailed descriptions of the effects achieved by the compounds described herein are evident in the examples, in particular in Figures 3, 4, 5 and 9 .

Additionally, the compounds described herein display toxicity characteristics that support its application as pharmaceutical agents. For a pharmaceutical agent to be viable for human use, the ratio of effect versus adverse effect is one critical factor in determining effective therapeutic application of a compound. The inventors have established that the compounds described herein reveal properties in both in vitro and in vivo systems that support a possible use in mammals, preferably human subjects. Further detailed descriptions of toxicity properties displayed by the compounds described herein are evident in the examples, in particular in Figures 2, 5, 7 and 8.

The compounds of the present invention, specifically inhibiting transcription of the S100A4 gene, and its mediated intracellular effects, are thus particularly useful as therapeutic agents, as they precisely target an underlying molecular mechanism that contributes to the development and progression of various diseases associated with the S100A4 protein, offering therapeutic strategies for treating conditions such as cancer. The compounds described herein may also be employed to develop additional compounds, based on the scaffolds and structures herein, to develop optimized therapeutic agents.

To the best knowledge of the inventors, no disclosure or suggestion is evident in the prior art that the compounds described herein would have beneficial pharmacokinetic properties and high efficacy as transcriptional inhibitors in the therapeutic applications according to the present invention. In light of the prior art, it would not have been possible for a person of ordinary skill to predict these surprisingly beneficial properties and their unexpected potency in inhibiting S100A4 displayed by the compounds described herein.

In one embodiment, the substituents of Formula 1 are as follows:
**X** is carbonyl -(C=O)- or sulfonyl -(SO₂)-,
**R¹** is a bicyclic heteroaryl group (preferably comprising N, S and/or O, more preferably an azole, thiazole or benzothiazol), optionally substituted with one or more of C1 to C5 alkyl (preferably C1 to C3 alkyl), halogen (preferably F, Cl and/or Br), nitro, alkyl sulfonyl (preferably C1 to C3), and
**R²** is phenyl, optionally substituted with one or more of C1 to C3 alkyl, halogen (preferably F, Cl and/or Br), or alkoxy (preferably OCH₂CH₃), wherein optionally multiple alkoxy substituents on R² form a fused bicyclic ring structure (preferably benzodioxanyl).

In one embodiment, the invention relates to a compound for use in the treatment of cancer according to **Formula 2** wherein 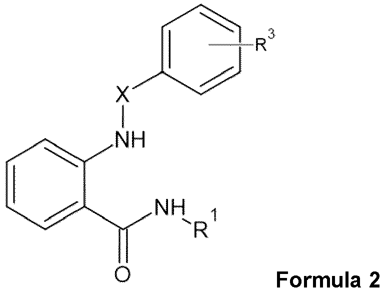 wherein
**X** is carbonyl -(C=O)- or sulfonyl -(SO₂)-,
**R¹** is a mono- or bicyclic heteroaryl group (preferably comprising N, S and/or O, more preferably an azole, thiazole or benzothiazol),

**R3** is one or more of C1 to C5 alkyl (preferably C1 to C3 alkyl), halogen (preferably F, Cl, Br and/or I), C1 to C5 alkoxy (preferably C1 to C3 alkoxy, wherein optionally two R³ groups form a ring structure fused with the adjacent phenyl), haloalkyl (preferably comprising F), hydroxyl, acyl.In one embodiment, the invention relates to a compound for use in the treatment of cancer according to **Formula 3** 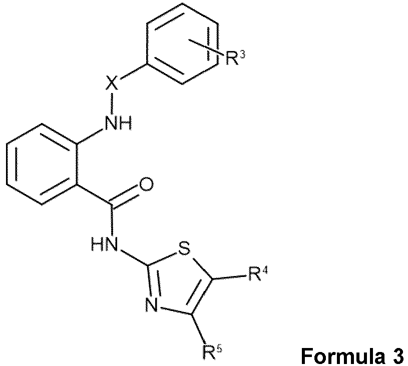 wherein
**X** is carbonyl -(C=O)- or sulfonyl -(SO₂)-,
**R3** is one or more of C1 to C5 alkyl (preferably C1 to C3 alkyl), halogen (preferably F, Cl, Br and/or I), C1 to C5 alkoxy (preferably C1 to C3 alkoxy, wherein optionally two R³ groups form a ring structure fused with the adjacent phenyl), haloalkyl (preferably comprising F), hydroxyl, acyl.
**R4** is H, C1 to C5 alkyl (preferably C1 to C4 alkyl), optionally connected to **R5** forming a (preferably aromatic) ring structure fused with the adjacent thiazole (preferably forming a benzothiazole), aryl (preferably phenyl), optionally substituted with one or more of C1 to C5 alkyl (preferably C1 to C3 alkyl), halogen (preferably F, Cl and/or Br), nitro, alkyl sulfonyl (preferably C1 to C3), alkoxy (preferably OCH₂CH₃), haloalkyl (preferably comprising F), hydroxyl, acyl, and
**R5** is H, C1 to C5 alkyl (preferably C1 to C4 alkyl), optionally connected to **R4** forming a (preferably aromatic) ring structure fused with the adjacent thiazole (preferably forming a benzothiazole), aryl (preferably phenyl), optionally substituted with one or more of C1 to C5 alkyl (preferably C1 to C3 alkyl), halogen (preferably F, Cl and/or Br), nitro, alkyl sulfonyl (preferably C1 to C3), alkoxy (preferably OCH₂CH₃), haloalkyl (preferably comprising F), hydroxyl, acyl.

In one embodiment, the invention relates to a compound for use in the treatment of cancer according to **Formula 4** 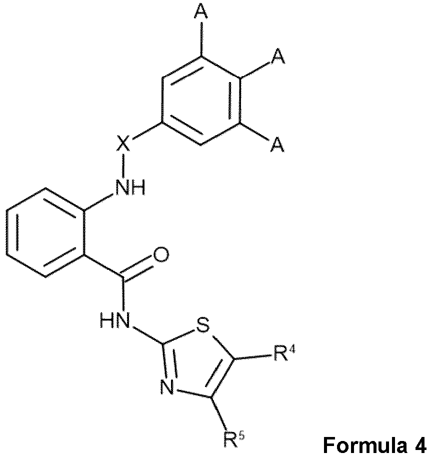 wherein
**X** is carbonyl -(C=O)- or sulfonyl -(SO₂)-,
**R4** is H, C1 to C5 alkyl (preferably C1 to C4 alkyl), optionally connected to **R5** forming a (preferably aromatic) ring structure fused with the adjacent thiazole (preferably forming a benzothiazole), aryl (preferably phenyl), optionally substituted with one or more of C1 to C5 alkyl (preferably C1 to C3 alkyl), halogen (preferably F, Cl and/or Br), nitro, alkyl sulfonyl (preferably C1 to C3), alkoxy (preferably OCH₂CH₃), haloalkyl (preferably comprising F), hydroxyl, acyl, and
**R5** is H, C1 to C5 alkyl (preferably C1 to C4 alkyl), optionally connected to **R4** forming a (preferably aromatic) ring structure fused with the adjacent thiazole (preferably forming a benzothiazole), aryl (preferably phenyl), optionally substituted with one or more of C1 to C5 alkyl (preferably C1 to C3 alkyl), halogen (preferably F, Cl and/or Br), nitro, alkyl sulfonyl (preferably C1 to C3), alkoxy (preferably OCH₂CH₃), haloalkyl (preferably comprising F), hydroxyl, acyl.
**A** can be the same or different, H, C1 to C5 alkyl (preferably C1 to C3 alkyl), halogen (preferably F, Cl, Br and/or I), C1 to C5 alkoxy (preferably C1 to C3 alkoxy), wherein optionally two A groups form a ring structure with the adjacent phenyl, hydroxyl, acyl.

In one embodiment, the invention relates to a compound for use in the treatment of cancer according to **Formula 5** 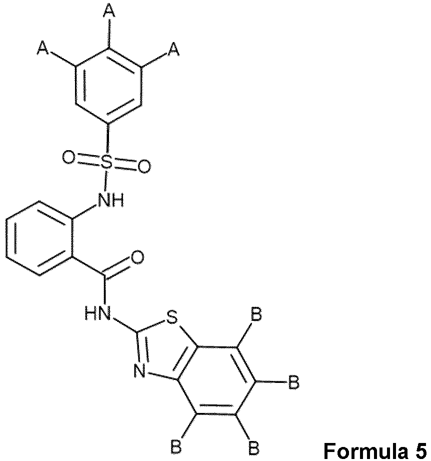 wherein
A can be the same or different, H, C1 to C5 alkyl (preferably C1 to C3 alkyl), halogen (preferably F, Cl, Br and/or I), C1 to C5 alkoxy (preferably C1 to C3 alkoxy), wherein optionally two A groups form a ring structure with the adjacent phenyl, hydroxyl, acyl.
B can be the same or different, H, C1 to C5 alkyl (preferably C1 to C3 alkyl), halogen (preferably F, Cl and/or Br), nitro, alkyl sulfonyl (preferably C1 to C3), alkoxy (preferably OCH₂CH₃), haloalkyl (preferably comprising F), hydroxyl, acyl.

In one embodiment the invention relates to a compound for use in the treatment of cancer according to **Formula 6** 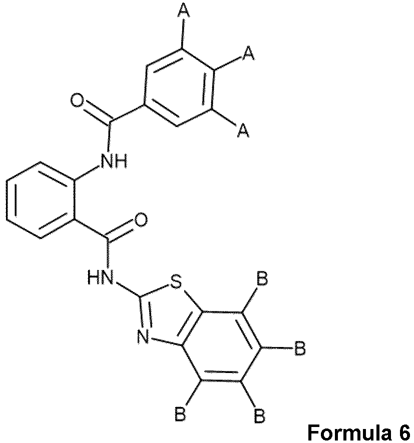 wherein
A can be the same or different, H, C1 to C5 alkyl (preferably C1 to C3 alkyl), halogen (preferably F, Cl, Br and/or I), C1 to C5 alkoxy (preferably C1 to C3 alkoxy), wherein optionally two A groups form a ring structure with the adjacent phenyl, hydroxyl, acyl.
B can be the same or different, H, C1 to C5 alkyl (preferably C1 to C3 alkyl), halogen (preferably F, Cl and/or Br), nitro, alkyl sulfonyl (preferably C1 to C3), alkoxy (preferably OCH₂CH₃), haloalkyl (preferably comprising F), hydroxyl, acyl.

In one embodiment, the compound is one of those shown below:

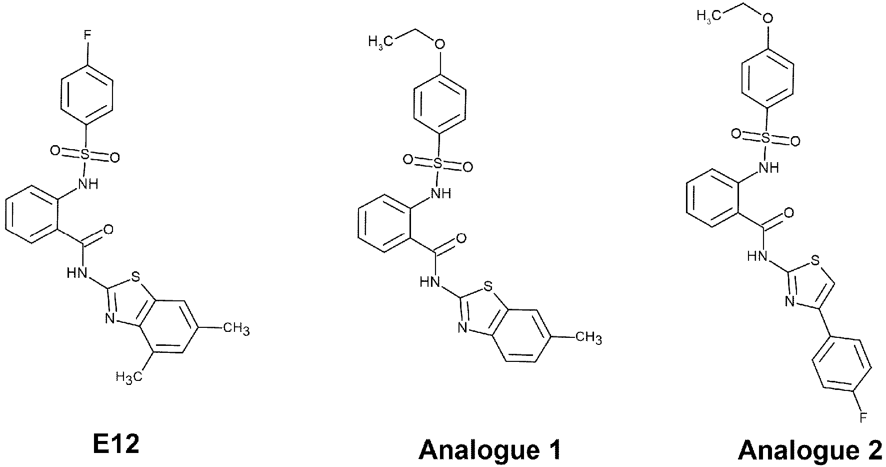

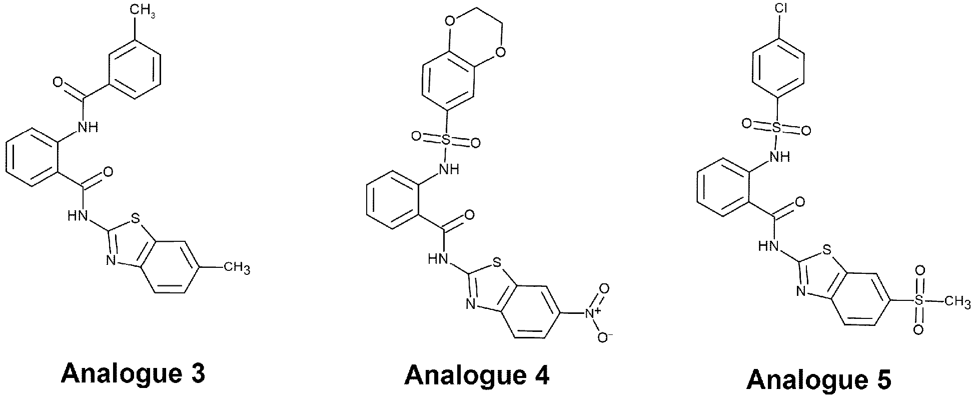

In one embodiment, the invention relates to a compound according to any one of Formulas 1, 2, 3, 4, 5 and 6 for use in the treatment of cancer, wherein the compound inhibits transcription of S100A4.

In one embodiment, the cancer comprises transcription of S100A4. In embodiments, the cancer comprises preferably S100A4 expression above levels in a non-cancerous cell. In embodiments, the cancer to be treated expresses S100A4 at detectable levels, preferably at levels above control levels. Suitable controls may relate to any non-cancerous cell, or other control In which S100 A4 is considered to be not expressed or expressed at negligible amounts.

A skilled person is capable of determining elevated levels of S100A4 using standard techniques. By way of example, a method may be employed selected from the group consisting of nucleic acid amplification methods, such as PCR, qPCR, RT-PCR, qRT-PCR or isothermal amplification, mass spectrometry (MS), luminescence immunoassay (LIA), radioimmunoassay (RIA), chemiluminescence- and fluorescence- immunoassays, enzyme immunoassay (EIA), enzyme-linked immunoassays (ELISA), luminescence-based bead arrays, magnetic beads based arrays, protein microarray assays, rapid test formats such as for instance immunochromatographic strip tests, rare cryptate assay, and automated systems/analyzers.

Elevated levels of S100A4 may be determined in comparison to an appropriate control. Control samples or patients may relate to a healthy control subject or samples therefrom, such as a subject with no detectable cancer or history of cancer. When considering the risk of metastasis, a control may also involve the use of samples from patients with cancer (for example those specific cancers described herein), but with no metastasis. As a positive control, samples from patients with elevated S100A4 expression and/or extensive metastasis may be used.

Elevated levels of S100A4 may be determined via nucleic acid and/or protein levels. Increased amounts of S100A4 protein and/or mRNA transcripts may be used to detect the levels of S100A4 in patients or control subjects. S100A4 is expressed in tumour tissue. Biopsy samples or removed tumors may be used to measure S100A4 levels as described herein. Furthermore, S100A4 protein, and/or S100A4 encoding nucleic acids, are detectable in bodily fluids of patients, such as urine or blood, or samples derived from blood such as blood plasma or serum, of patients. Specific methods, useful in the invention but not limiting to the invention, for identifying elevated levels of S100A4 are described in more detail below.

The treatment of patients with elevated levels of S100A4 may, in some embodiments, comprise a combined method incorporating diagnosis of a cancer on the basis of elevated S100A4 levels, for example an elevation in S100A4 transcript levels, and subsequent treatment with the compounds described herein.

Patients who have been diagnosed with cancer or with increased risk of developing cancer or metastases on the basis of S100A4 levels can now be treated directly via interrupting S100A4 function. The invention therefore enables efficient therapeutic approaches for those patients with elevated S100A4 levels. The inhibitors of the present invention enable effective treatment according to a novel patient stratification strategy. Those patients without elevated S100A4 levels are typically not selected for treatment with the compounds disclosed herein, thereby making administration of the compounds described herein targeted towards a population of patients with elevated S100A4 levels who will likely respond to the treatment, thereby saving time and financial cost of treating non-responders without elevated S100A4 levels.

The group of patients defined by elevated S100A4 levels compared to a suitable control group represents a patient group that is defined by a clear pathological and physiological criterion that is directly related to disease occurrence and progression.

In one embodiment, the invention relates to a compound according to any one of Formulas 1, 2, 3, 4, 5 and 6, for use in the treatment of a cancer comprising an abberrant Wnt-signaling compared to a non-cancerous cell.

The Wnt/β-catenin signaling pathway target gene S100A4 is overexpressed in many different types of cancer, such as gallbladder, bladder, breast, esophageal, gastric, pancreatic, hepatocellular, non-small cell lung and especially colorectal cancer. Increased expression of S100A4 is strongly associated with aggressiveness of a tumor, its ability to metastasize and poor survival in patients. Inhibition of S100A4 has also been shown to lead to inhibit S100A4-induced cell migration and invasion as well as cell proliferation and colony formation in vitro.

In one embodiment the invention relates to a compound according to any one of Formulas 1, 2, 3, 4, 5 and 6, for use in the treatment and/or prevention of cancer metastasis. In embodiments, the compounds of the invention are shown to inhibit metastatic cancer cell motility, especially of metastatic cell motility relying on, associated with and/or driven by S100A4 expression.

As is shown in the examples below, the beneficial properties are evidenced via both in vitro and in vivo models. The in vitro approach employed uses a wound healing assay with the HCT116 human colon cancer cell line. HCT116 cells are endogenously positive for MACC1 and S100A4 expression at the mRNA and protein level. An efficacy against cell motility may be quantified based on the direct effect on reduced cell motility of HCT116 cells observed in the wound healing assay. Cell motility is a fundamental cellular behaviour that contributes directly to the pathology of metastasis. By exhibiting an effect on cell motility, as evidenced in the wound healing assay, the therapeutic setting of treating and/or preventing metastasis is supported.

In one embodiment, the cancer to be treated is a solid tumor, or selected from the group consisting of gastrointestinal, colorectal, gastric, esophageal, pancreatic, hepatocellular, biliary, lung, nasopharyngeal, renal, bladder, ovarian, brain, bone, head and neck, prostate, melanoma and breast cancer.

Solid tumors have been analyzed previously by the inventors and others and characterized for their responsiveness to other inhibitors of the Wnt/β-catenin signaling pathway and/or other inhibitors of MACC1. The solid tumors listed above are known to respond to e.g. niclosamide. The solid tumors listed above are known to exhibit expression of MACC1 and/or show active Wnt/β-catenin signaling, preferably evident via S100A4 expression.

In one embodiment, the compounds described herein are for use in the treatment of a solid tumor or a cancer selected from the group consisting of colorectal, gastric, esophageal, pancreatic, lung, bladder, ovarian and prostate cancer.

In one embodiment, the compounds described herein are for use in the treatment and/or prevention of colorectal cancer metastasis.

In another aspect, the invention relates to a pharmaceutical composition for use in the treatment of cancer, comprising one or more compounds according to any one Formulas 1, 2, 3, 4, 5 and 6 or a pharmaceutically acceptable salt thereof, and a pharmaceutically acceptable carrier.

In another aspect, the invention relates to the use of a compound described herein in an in vitro method to inhibit S100A4 activity, preferably transcription, in an in vitro, in vivo or ex vivo assay.

In another aspect, the invention relates to the use of a compound described herein as a lead compound to generate further chemical derivatives for assessment as candidates as inhibitors of S100A4, using, for example, one or more of the assays described herein.

All features described in the present specification may be employed to define any other embodiment or aspect of the invention, for example, features used to describe the medical use of one compound may be used to describe a medical use of another compound, and vice versa. Features used to describe any one or more compounds may be combined with any one or more of the medical uses of the invention. Similarly, structural features of the compounds may be used to describe the methods of the invention, other compounds of the invention or pharmaceutical composition and vice versa, according to the understanding of a skilled person.

### DETAILED DESCRIPTION OF THE INVENTION

The invention relates to chemical compounds and their medical use as transcriptional inhibitors of S100A4, preferably for the treatment of cancer. The invention further relates to a pharmaceutical composition comprising the compounds, and use of the compounds or composition in the treatment of cancer, a tumor disease, such as a solid tumor, and/or for the treatment and/or prophylaxis of tumor metastasis.

### General terms:

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of' and "consisting essentially of".

Thus, the terms "comprising"/"including"/"having" mean that any further component (or likewise features, integers, steps and the like) can/may be present. The term "consisting of' means that no further component (or likewise features, integers, steps and the like) is present.

The term "consisting essentially of' or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

Thus, the term "consisting essentially of' means those specific further components (or likewise features, integers, steps and the like) can be present, namely those not materially affecting the essential characteristics of the composition, device or method. In other words, the term "consisting essentially of" (which can be interchangeably used herein with the term "comprising substantially"), allows the presence of other components in the composition, device or method in addition to the mandatory components (or likewise features, integers, steps and the like), provided that the essential characteristics of the device or method are not materially affected by the presence of other components.

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts. As used herein, the "patient" or "subject" may be a vertebrate. In the context of the present invention, the term "subject" includes both humans and animals, particularly mammals, and other organisms.

### Chemical compounds

With respect to the chemical compounds described herein, the term "alkyl" refers to a branched or unbranched hydrocarbon group of preferably 1 to 5 carbon atoms, such as methyl, ethyl, n-propyl, isopropyl, n-butyl, isobutyl, sec-butyl, pentyl, and the like. Preferred alkyl groups have 1-5 carbon atoms, more preferably 1-4 or 1-3, 2 or 1 carbon atoms. In one embodiment the alkyl group may be saturated. In one embodiment the alkyl group may be unsaturated when the alkyl group comprises three or more carbon atoms. In one embodiment any one or more of the alkyl groups described herein may be "substituted alkyls", wherein one or more hydrogen atoms of the alkyl are substituted with a substituent such as halogen (preferably F, Cl and/or Br), cycloalkyl (preferably cyclopropane and/or cyclobutane), hetero cycloalkyl (preferably oxetane), alkoxy, hydroxyl, aryl, carboxyl, OR and/or SR, wherein R is preferably H, alkyl, cycloalkyl, hetero cycloalkyl, alkoxy, aryl, heteroaryl or amine.

The term "alkoxy" refers to a straight, branched or cyclic hydrocarbon configuration and combinations thereof, including preferably 1-7 carbon atoms, more preferably 1-6, 1-5, 1-4 or 1-3 carbon atoms, that include an oxygen atom at the point of attachment (such as O-alkyl). An example of an "alkoxy group" is represented by the formula -OR, or -ROR (such as -CH₂OR), where R can be an alkyl group, optionally substituted with halogen, aryl (also referred to as aryl oxy), cycloalkyl, halogenated alkyl. Suitable alkoxy groups include methoxy, ethoxy, n-propoxy, i-propoxy, n-butoxy, i-butoxy, sec-butoxy, cyclohexyloxy, and the like.

The term "aryl" refers to any carbon-based aromatic group including, but not limited to, benzene, naphthalene, and the like. The term "aromatic" also includes "heteroaryl group," which is defined as an aromatic group that has at least one heteroatom incorporated within the ring of the aromatic group. Examples of heteroatoms include, but are not limited to, nitrogen, oxygen, sulfur. The aryl group can be substituted with one or more groups including, but not limited to, alkyl, aryl, halogen, nitro, hydroxy, carboxylic acid, or alkoxy, or the aryl group can be unsubstituted.

The term "heteroaryl" is understood to unsaturated (heteroaryl) hydrocarbon rings containing from 3 to 15 carbon atoms in a mono- or bicyclic, fused, bridged or spirocyclic ring in which 1 to 5 carbon atoms of the 3 to 15 ring carbon atoms are replaced by heteroatoms such as nitrogen, oxygen or sulfur in which further the heteroatoms can be oxidized, for example N=O, S=O, SO₂. Non-limiting examples of heterocycles are acridinyl, azaindole (1H-pyrrolopyridinyl), azabenzimidazolyl, azaspirodecanyl, azepinyl, azetidinyl, aziridinyl, benzimidazolyl, benzofuranyl, dihydrobenzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 3,3-dioxo[1,3,4]oxathiazinyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,3-triazolyl, 1,2,4-triazolyl, 1,2,5-triazolyl, 1,3,4-triazolyl and xanthenyl.

"Carbonyl" refers to a group of the formula -C(O)-. Carbonyl-containing groups include any substituent containing a carbon-oxygen double bond (C=O), including acyl groups, amides, carboxy groups, carboxylic acid esters (also termed alkoxy carbonyl), ureas, carbamates, carbamate ester, carbonates, carboxamides, imides and ketones and aldehydes, such as substituents based on -COR or -RCHO where R is an aliphatic group, heteroaliphatic group, alkyl, heteroalkyl, hydroxyl, or a secondary, tertiary, or quaternary amine, phenyl, a substituted phenyl (substituted with, for example, halogen, C1-C3 alkyl, alkoxy, amine), carboxyl, alkoxycarbonyl, amine, aryl.

The term "halogen" preferably refers to fluorine, chlorine, bromine or iodine. If the term is used for a residue, "halogen" means, for example, a fluorine, chlorine, bromine or iodine atom.

According to the invention, "alkylsulfonyl" or "arylsulfonyl" means an alkyl group or aryl group containing a sulfonyl group (-SO₂-). This group is also known as the sulfone group. Sulfonyl compounds belong to the organic derivatives of oxygen acids of sulfur, wherein the two organic residues are bonded directly to the sulfur atom.

The term "hydroxyl" is represented by the formula -OH.

Optionally substituted groups, such as "optionally substituted alkyl," refers to groups, such as an alkyl group or an aryl alkyl group, that when substituted, have from 1-5 substituents, typically 1, 2 or 3 substituents, preferably selected from hydroxy and alkoxy.

Optionally substituted alkyl groups include, by way of example, haloalkyl groups, such as fluoroalkyl groups, including, without limitation, trifluoromethyl groups. These potential optional substituents apply to any group of the formula disclosed herein where an optional substituent is recited. Preferable optional substituents are hydroxyl, alkyl, alkoxy, carbonyl, alkoxycarbonyl, NO₂, amine.

The term "sulfonyl" is represented by the formula -SO₂R.

The terms "amine sulfoxide", "sulfonamide", "sulfonamide amine" are preferably selected from the groups -N(R)-S(O)ᵤ-R, wherein u is 1 or 2, or -S(O)ᵥ-N(R)-R, wherein v is 1 or 2, preferably from the groups -NHSO₂CH₃, -SO₂NHCH₃, -NHSO₂N(CH₃)₂).

For the definitions above, preferably the terms R, R' are independently selected from the group of H, alkyl, alkylhalo, alkoxy, amine or any of the embodiments described above, and wherein X is halogen. The terms R, R' also comprise the possibility of any given group being appended to R.

The term "nitro" refers to an NO₂ group.

The term "bicyclic or tricyclic ring structure" refers to a structure comprising two or three interconnected cyclic structures, respectively. These ring structures may be either fused (sharing two or more atoms) or bridged (connected by a single atom or a chain of atoms).

Where reference is made to "C1 to C7, C1 to C5, C1 to C3" alkyl, cycloalkyl, alkoxy, aryl, or the like, the number of carbon atoms C1 to C7 preferably refers to each of the substituents mentioned, although in some embodiments the shorter substituents of C1 to C5 or C1 to C3 apply to the alkyl, cycloalkyl and/or alkoxy groups, whereby aryl may remain preferably C3 to C6, such as C6 phenyl.

The term "bicyclic or tricyclic ring structure" refers to a group comprising two or three 4- to 8-membered cyclic, optionally aromatic, ring structures, optionally comprising one or more heteroatoms such as one or more of N, O and/or S. Within the "bicyclic or tricyclic ring structure" the two or three ring structures are attached to each other by two of their adjacent atoms (condensed ring structure), by one atom (spiro ring structure) and/or one or more bridging atoms (bridge ring structures). An example of a bicyclic ring structure is indole. Examples for further bicyclic or tricyclic ring structures can for example be found in WO 2008/040332, WO 2011/003626, WO 2013/030111, WO2016/092069 and WO2019/162524. The bicyclic or tricyclic ring structure may optionally be substituted such as by halogen (preferably fluorine, chlorine, bromine and iodine), OH, CN, SH, amine (preferably NH₂, NHCH₃), NO₂, or -alkyl, with alkyl optionally substituted with one or more substituents such as the substituents listed in this paragraph.

The term "alpha position" refers to the first carbon atom directly attached to a functional group, such as a carboxyl group, ester group, or amide group. For an alkyl carboxamide, the alpha position refers to the carbon atom directly attached to the carboxamide group. For an alkyl carboxyl ester, the alpha position refers to the carbon atom directly attached to the carboxyl ester group. For an alkyl carboxyl, the alpha position refers to the carbon atom directly attached to the carboxyl group. The term "substituted in the alpha position" refers to a substituent attached to the alpha position of a functional group such as in the alpha position of an alkyl carboxyl, alkyl carboxamide or alkyl carboxyl ester.

Protected derivatives of the disclosed compound also are contemplated, for example for use in the synthesis of the disclosed compounds. A variety of suitable protecting groups for use with the disclosed compounds are disclosed in Greene and Wuts Protective Groups in Organic Synthesis; 3rd Ed.; John Wiley & Sons, New York, 1999. In general, protecting groups are removed under conditions which will not affect the remaining portion of the molecule. These methods are well known in the art and include acid hydrolysis, hydrogenolysis and the like.

Particular examples of the presently disclosed compounds may or may not include one or more asymmetric centers (chiral centers); thus these compounds can exist in different stereoisomeric forms such as enantiomers or diastereoisomers such as epimers. Accordingly, compounds and compositions may be provided as individual pure stereoisomeric forms or as stereoisomeric mixtures, including racemic mixtures. The term "ambo" refers to a molecule with two or more chiral elements present as a mixture of diastereoisomers due to the undefined configuration of at least one chiral element of the molecule.

The compounds of the invention may also exist in various polymorphous forms, for example as amorphous and crystalline polymorphous forms. All polymorphous forms of the compounds of the invention belong within the framework of the invention and are a further aspect of the invention.

The compound of the invention may also comprise deuterium replacing hydrogen. This replacement may in some circumstances lead to improved metabolic stability (Nature Reviews Drug Discovery 15, 219-221 (2016)).

It is understood that substituents and substitution patterns of the compounds described herein can be selected by one of ordinary skill in the art to provide compounds that are chemically stable and that can be readily synthesized by techniques known in the art and further by the methods set forth in this disclosure.

### Inhibitors:

According to the present invention, an "inhibitor" in the context of "a transcriptional inhibitor of S100A4" or "an inhibitor of S100A4" is considered any agent, substance, compound, molecule or other means leading to a slowing, repressing, blocking or other interfering or negative action on the activity, function, expression of or signaling caused by the named target.

For example, an inhibitor of S100A4 may affect S100A4 protein function, S100A4 expression (transcription or translation) and/or S100A4-mediated signaling, either directly or indirectly. The inhibitors as described herein may also be termed "agents". References to the "agents" in the context of the methods described herein are to be understood as the inhibitor of S100A4 Preferred inhibitors are those described herein.

### S100A4:

One major target that is linked to metastasis formation is S100 calcium binding protein A4 (S100A4), an 11 kDa protein, originally identified as metastasin 1(MTS1). S100A4 is also known as: 18A2, 42A, CAPL, FSP1, MTS1, P9KA, PEL98. "S100A4" as used herein refers to the S100 calcium binding protein A4 in Homo sapiens according to the protein ID: NP_002952 of the NCBI database. The S100A4 gene encodes a 101 amino acid protein recorded under GenelD: 6275. Appropriate means for detecting S100A4 encoding nucleic acids or S100A4 proteins are provided herein.

S100A4 is overexpressed in many different types of cancer such as gallbladder, bladder, breast, esophageal, gastric, pancreatic, hepatocellular, non-small cell lung and especially colorectal cancer. Increased expression of S100A4 is strongly associated with aggressiveness of a tumor, its ability to metastasize and poor survival in patients. However, S100A4 itself is not tumorigenic because transgenic mice overexpressing S100A4 do not develop tumors per se. But, when S100A4 transgenic mice are crossed with mice demonstrating spontaneous tumor formation, it leads to aggressive tumor growth and metastasis. Moreover, S100A4-null mice injected with highly metastatic mouse mammary carcinoma cells show no metastases. These observations suggest that S100A4 is essential for the process of metastasis formation.

S100A4 plays a major role in cellular processes such as migration, invasion, adhesion and angiogenesis, which form the basis for metastasis formation. For instance, S100A4 increases cell motility by interacting with proteins from the cytoskeleton, such as the heavy chain of non-muscle myosin II (MYH9). Moreover, S100A4 participates in cell adhesion by interaction with protein tyrosine phosphatase receptor type F (PTPRF) interacting protein, binding protein 1 (PPFIBP1; also known as liprin β-1) and promotes cell invasion and angiogenesis via upregulation of matrix metalloproteinase (MMPs).

Inhibitors of S100A4 can be determined using established and routine techniques. For example, any given assay may be employed where a substance suspected of being a S100A4 inhibitor is assessed and compared to an appropriate control, using the commercially available technologies described herein (e.g. ELISA kits available from Aviva Systems Biology OKCA02438 or LifeSpan Biosciences LS-F9180-1) or the technology of the cited prior art (e.g. Sack et al., J Natl Cancer Inst. 2011 Jul 6;103(13):1018-36; Garrett et al, Biochemistry. 2008 Jan 22; 47(3): 986-996). S100A4 protein or activity may be assessed or levels of S100A4 transcripts may be determined in a cellular assay or from a biological sample, in order to determine reduced activity or amounts of S100A4 induced by treatment of any given substance (candidate inhibitor).

S100A4 was also shown to have an impact on pathology related to fibrotic, inflammatory, pulmonary, neurological and cardiovascular diseases [60-64] where it is involved in promoting cell motility, invasion, angiogenesis and inflammation. In fibrosis it has been described as a damage-associated molecular pattern which evokes signalling cascades involving downstream mediators facilitating extracellular matrix deposition and myofibroblast generation. S100A4 can also play a role in persistent activation of myofibroblasts.

### Determination of S100A4 levels in patients:

The determination of elevated S100A4 levels in patient samples has been described in the art. These methods may be applied in the present invention.

In a preferred embodiment the invention relates to the treatment of a group of patients that are identified by increased expression of S100A4, preferably whereby the cancerous cells show increased expression of S100A4. S100A4 expression can be determined using any commonly known method in the art, such as RT-PCR for analysis of increased expression of S100A4 transcript or immunological based methods such as western blot or ELISA for detection of increased expression of S100A4 protein, or any other diagnostic tools that can provide relative measurements of S100A4 expression in comparison to "normal" or "healthy" or "low risk" cells.

As is disclosed in US20140294957A1, total RNA can be isolated from 4 × 10⁵ cells plated in a 6-well-plates 24 hour before cells are lysed with Trizol Reagent (Invitrogen). Alternatively, RNA can be isolated from blood samples, or tumor tissue samples, and treated accordingly. For example, RNA is extracted with Trizol RNA extraction reagent (Invitrogen) according to the manufacturer's instructions. Quantification of the RNA concentration is performed with Nanodrop (Peqlab) and 50 ng total RNA is reverse transcribed with random hexamers in 10 mM MgCl2; 1x RT-buffer, 250 µ M pooled dNTPs, 1 U per µL RNAse inhibitor and 2.5 U per µL MuLV reverse transcriptase (all Applied Biosystems). Reaction occursat 42°C for 15 minutes, 99°C for 5 minutes and subsequent cooling at 5°C for 5 minutes. The cDNA product is amplified in a total volume of 10 µL in 96-well-plates in the LightCycler 480 (Roche) using the following conditions: 95°C, 10 minutes, followed by 45 cycles of 95°C for 10 seconds, 61°C for 30 seconds and 72°C for 4 seconds, with appropriate primers, such as those disclosed in US20140294957A1, which is hereby incorporated in entirety by reference. For cDNA quantification of the housekeeping gene glucose-6-phosphate dehydrogenase (G6PD) the hG6PDH Roche Kit (Roche Diagnostics, Mannheim, Germany) is used according to standard instructions. Data analysis is performed with e.g. LightCycler^{®} 480 Software release 1.5.0 SP3. For each qRT-PCR reaction a mean of the duplicate is calculated. Each mean value of the expressed gene is normalized to the respective mean amount of the G6PD cDNA.

### Additional diagnostic approaches in determining S100A4 levels:

The invention further relates to kits, the use of the kits and methods for determining S100A4 levels. Such means can also be used to determine S100A4 levels during functional testing of inhibitors. The herein provided definitions, e.g. provided in relation to the methods, also apply to the kits of the invention. In particular, the invention relates to the use of kits for determining S100A4 levels, wherein said kit comprises
- detection reagents for determining the level of S100A4, and
- reference data for determining elevated levels of S100A4, in particular reference data for threshold or cut-off value(s), wherein said reference data is preferably stored on a computer readable medium and/or employed in the form of computer executable code configured for comparing the determined levels of S100A4 with the threshold or cut-off value(s).

As used herein, "reference data" comprise reference level(s) of S100A4, preferably from a healthy patient. The levels of S100A4 in the sample of the subject can be compared to the reference levels comprised in the reference data of the kit. The reference data can also include a reference sample to which the level of S100A4 is compared. The reference data can also include an instruction manual how to use the kits of the invention.

The kit may additionally comprise items useful for obtaining a sample, such as a blood sample, for example the kit may comprise a container, wherein said container comprises a device for attachment of said container to a canula or syringe, is a syringe suitable for blood isolation, exhibits an internal pressure less than atmospheric pressure, such as is suitable for drawing a pre-determined volume of sample into said container, and/or comprises additionally detergents, chaotropic salts, ribonuclease inhibitors, chelating agents, RNAse inhibitor proteins, and mixtures thereof.

As used herein, the "detection reagent" or the like are reagents that are suitable to determine the herein described marker(s), e.g. of S100A4. Such exemplary detection reagents are, for example, nucleic acid primers for amplifying S100A4 encoding nucleic acid sequences. Such primers might be used in a nucleic acid amplification reaction, e.g. a PCR method. Such exemplary detection reagents are, for example, ligands, e.g. antibodies or fragments thereof, which specifically bind to the S100A4 peptide or epitopes of the herein described marker(s). Such ligands might be used in immunoassays.

The term "nucleic acid amplification reaction" refers to any method comprising an enzymatic reaction, which allows the amplification of nucleic acids. One preferred embodiment of the invention relates to a polymerase chain reaction (PCR). Another preferred embodiment relates to real time PCR (RT-PCR) or quantitative RT-PCR (qRT-PCR), as it allows the quantification of the amplified target in real-time. The term "real-time PCR" is intended to mean any amplification technique which makes it possible to monitor the progress of an ongoing amplification reaction as it occurs (i.e. in real time). Data is therefore collected during the exponential phase of the PCR reaction, rather than at the end point as in conventional PCR. Measuring the kinetics of the reaction the early phases of PCR provides distinct advantages over traditional PCR detection. In real-time PCR, reactions are characterized by the point in time during cycling when amplification of a target is first detected rather than the amount of target accumulated after a fixed number of cycles. The higher the starting copy number of the nucleic acid target, the sooner a significant increase in fluorescence is observed. Traditional PCR methods may also be applied, and use separation methods, such as agarose gels, for detection of PCR amplification at the final phase of or end-point of the PCR reaction. For qRT-PCR no post-PCR processing of the unknown DNA sample is necessary as the quantification occurs in real-time during the reaction. Furthermore, an increase in reporter fluorescent signal is directly proportional to the number of amplicons generated. A skilled person is aware of the reagents and methods necessary for carrying out such detection.

### MACC1:

Metastasis remains a major challenge in colorectal cancer (CRC) management. One recent key finding in understanding molecular pathogenesis of CRC metastasis is the identification of the gene MACC1. MACC1 has been discovered as prognostic biomarker for metastasis and metastasis-free survival in CRC. Meanwhile MACC1 is confirmed as a decisive driver for tumorigenesis and metastasis in various other solid cancers.

"MACC1" as used herein refers to the metastasis associated in colon cancer 1 gene in Homo sapiens according to the Gene ID: 346389 of the NCBI database. The gene encodes an 852 amino acid protein recorded under GenBank ID: AA137091. Appropriate means for detecting MACC1 encoding nucleic acids or proteins are provided in the examples below.

Inhibitors of MACC1 can be determined using established and routine techniques. For example, any given assay may be employed where a substance suspected of being a MACC1 inhibitor is assessed and compared to an appropriate control, using the commercially available technologies described herein (e.g. ELISA kits available from Aviva Systems Biology OKCD09378 or Biomatik EKU05926) or the technology of the cited prior art (e.g. Juneja, Kobelt et al. PLoS Biol. 2017 Jun 1;15(6)). MACC1 protein or activity may be assessed or levels of MACC1 transcripts may be determined in a cellular assay or from a biological sample, in order to determine reduced activity or amounts of MACC1 induced by treatment of any given substance (candidate inhibitor).

### Wnt/β-catenin signaling pathway:

As used herein, the terms "Wnt/β-catenin signaling pathway" and "Wnt-signaling" or "Wnt-signaling pathway" may be used interchangeably.

Colorectal cancer is often associated with activation of the Wnt/β-catenin signaling pathway and high expression of the metastasis-inducing gene S100A4. In addition to its function in the cell-cell adhesion, β-catenin is also an important mediator of the canonical Wnt signalling pathway. When no Wnt signalling occurs, two scaffolding proteins, the tumour suppressor APC and AXIN, form the so-called destruction complex with β-catenin, which facilitates the sequential phosphorylation of β-catenin by CKI and GSK3-β at the amino-terminus. The phosphorylations recruit the F box/WD repeat protein β-TrCP-containing E3 ubiquitin ligase, which marks β-catenin for proteosomal degradation. The Wnt signalling pathway is activated by binding of the Wnt ligand to the Frizzled transmembrane receptor, a serpentine receptor with an amino-terminal cysteine-rich domain. The complex then interacts with a single-pass transmembrane protein of the LDL receptor family (LRP5/6). It is not clear how the FRZ/LRP complex regulates the kinase activity of the destruction complex. However, it is suggested that the activity axin/GSK3-β is inhibited by a mechanism involving the interaction of axin with LRP5/6 or the action of the axin binding molecule Dishevelled (DSH). Upon Wnt signaling activation, the destruction complex is deactivated and the unphosphorylated β-catenin translocates to the nucleus where it binds to the amino-terminus of the Tcf/Lef (T cell factor/lymphoid enhancer factor) family of DNA-binding proteins and activates the transcription of target genes. The Tcf/Lef proteins repress target genes in the absence of β-catenin, but transform into transcriptional activators once they bind to β-catenin.

The relevance of the Wnt pathway for cancer cells is indicated by the high percentage of mutations occurring in the genes of the Wnt pathway. For example, over 90% of colorectal cancers bear mutations that result in Wnt pathway activation. These mutations generally affect β-catenin phosphorylation and stability, hindering its degradation via the ubiquitin pathway. Non-phosphorylated β-catenin accumulates in the cytoplasm, is transported to the nucleus, and interacts with TCF family transcription factors to control target genes. Nuclear β-catenin accumulation has been associated with late stages of tumor progression and development of metastases, and the presence of mutated β-catenin is associated with aggressive tumor growth and poor prognosis.

As one example, S100A4 is considered a marker molecule or read-out for Wnt/β-catenin signaling. Other read-out molecules, by which their levels are indicative of Wnt/β-catenin signaling inhibition, are, without limitation, c-myc, MMP-7, -9 or-13.

Wnt/beta-Catenin Signaling and Small Molecule Inhibitors have been described in detail in Voronkov et al. (Current Pharmaceutical Design, 2013, 19, 634-664).

Inhibitors of the Wnt/β-catenin signaling pathway relate to Niclosamide, XAV939, IWR1, IWP-1, IWP-2, JW74, JW55, Okadaic acid, Tautomycin, SB239063, SB203580, ADP-HPD, 2-[4-(4-fluorophenyl)piperazin-1-yl]-6-methylpyrimidin4(3H)-one, PJ34, Cambinol, Sulindac, 3289-8625, J01-017a, NSC668036, Filipin, IC261, PF670462, Bosutinib, PHA665752, Imatinib, ICG-001, Ethacrynic acid, PKF115-584, PNU-74654, PKF118-744, CGP049090, PKF118-310, ZTM000990, BC21, GDC-0941, Rp-8-Br-cAMP.

Inhibitors of the Wnt/β-catenin signaling pathway can be determined using established and routine techniques. For example, QIAGEN provides a broad range of assay technologies for assessing WNT signaling research that enables analysis of gene expression and regulation, epigenetic modification, genotyping, and signal transduction pathway activation. Any given assay may be employed where a substance suspected of being a Wnt/β-catenin signaling pathway inhibitor is assessed and compared to an appropriate control, using the commercially available technologies described herein or the technology of the cited prior art (e.g. Sack et al. Mol Biol Cell. 2011 Sep;22(18):3344-54; Sack et al., J Natl Cancer Inst. 2011 Jul 6;103(13):1018-36).

### Cancer:

The invention relates to compounds for the treatment of cancer or cancer-like disorders, such as a cell proliferative disorder or cancer metastasis. The terms "cancer", "proliferative disorder" or "cellular proliferative disorder" may be understood interchangeably and refer to any disorder in which the proliferative capabilities of the affected cells is different from the normal proliferative capabilities of unaffected cells. An example of a cell proliferative disorder is neoplasia. Malignant cells develop as a result of a multistep process. The term "cancer", tumor disease" or "malignant tumor" may refer to a tumor or hematopoietic disease no longer under normal cellular growth control. The term "cancerous cell" includes a cell afflicted by any one of the cancerous conditions provided herein. The term "carcinoma" refers to a malignant new growth made up of epithelial cells tending to infiltrate surrounding tissues, and to give rise to metastases.

A cell proliferative disorder as described herein may be a neoplasm, commonly referred to as a tumor or tumour. Such neoplasms are either benign or malignant. The term "neoplasm", "tumor" or "tumour" refers to a new, abnormal growth of cells or a growth of abnormal cells that reproduce faster than normal. A neoplasm creates an unstructured mass (a tumor) which can be either benign or malignant. The term "benign" refers to a tumor that is noncancerous, e.g. its cells do not proliferate or invade surrounding tissues.

In another aspect, the invention provides a method of preventing, treating, and/or managing a solid tumor in a patient, the method comprising administering to a patient in need thereof a prophylactically effective regimen or a therapeutically effective regimen, the regimen comprising administering to the patient a compound of the invention, or a pharmaceutically acceptable salt thereof, wherein the patient has been diagnosed with a solid tumor. In particular embodiments of this aspect, the solid tumor is fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney-cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, or retinoblastoma.

### Metastasis:

In one embodiment, the invention relates to compounds for use in the treatment and/or prophylaxis of tumor metastasis, preferably by reducing cellular motility of cancerous cells. As used herein, the term "metastasis" refers to the spread of cancer cells from the place where they first formed to another part of the body.

Metastasis may be considered a multistep process where tumor cells disseminate from the primary tumor and colonize distant organs. The steps include, without limitation, tumor cell invasion of basement membranes and the surrounding tissue, intravasation into blood vessels, survival there, extravasation and/or growth at different organ sites. To achieve these steps, precise coordination of cell movement and matrix remodeling are required and can be interrupted by an anti-metastatic agent. Determining an effect against cancer metastasis may therefore employ determining an inhibitory effect against one or more of cell invasion, i.e. invasion of basement membranes and the surrounding tissue, cell motility or cell movement, invasion into a blood or lymphatic vessel (intravasation), and/or leakage of cancer cells from a blood or lymphatic vessel (extravasation). According to the present invention reduced cell motility is preferably assessed.

Models for assessing an effect against metastasis are disclosed herein and are known to a person skilled in the art. The in vitro approach employed preferably uses a wound healing assay with the HCT116 human colon cancer cell line. An effect on metastasis is quantified based on the direct effect on reduced cell motility of the HCT116 cells observed in the wound healing assay.

Effects may also be observed for reducing cancer cell motility, migration and/or invasion. In some embodiments, cancer metastasis can be distinguished from cancer proliferation, thereby representing a distinct medical use. In some embodiments, cell proliferation and cell metastasis are both reduced.

As used herein "cell migration" or "motility" is intended to mean movement of a cell towards a target, such as a chemical or physical signal. Cells often migrate in response to external signals, including chemical signals and mechanical signals. Cancer cells are capable of migrating, and exhibit cell motility, which are characteristics in metastatic cancer and may therefore be targeted to treat and/or reduce or reduce the risk of metastasis. Chemotaxis is one example of cell migration or motility regarding response to a stimulus. In vitro chemotaxis assays such as Boyden chamber assays may be used to determine whether cell migration occurs for any given cell.

For example, cancer cells may be purified and analyzed. Chemotaxis assays (for example according to Falk et al., 1980 J. Immun. Methods 33:239-247) can be performed using plates where a particular chemical signal is positioned with respect to the cells of interest and the transmigrated cells then collected and analyzed. For example, Boyden chamber assays entail the use of chambers isolated by filters, used as tools for accurate determination of chemotactic behavior. The pioneer type of these chambers was constructed by Boyden (Boyden (1962) "The chemotactic effect of mixtures of antibody and antigen on polymorphonuclear leucocytes". J Exp Med 115 (3): 453). The motile cells are placed into the upper chamber, while fluid containing the test substance is filled into the lower one. The size of the motile cells to be investigated determines the pore size of the filter; it is essential to choose a diameter which allows active transmigration. For modelling in vivo conditions, several protocols prefer coverage of filter with molecules of extracellular matrix (collagen, elastin etc.) Efficiency of the measurements can be increased by development of multiwell chambers (e.g. NeuroProbe), where 24, 96, 384 samples are evaluated in parallel. Advantage of this variant is that several parallels are assayed in identical conditions. Methods such as this can be used to assess whether any given cancer cell type exhibits cell motility or migration, i.e. a tumor samples could be obtained, the cells cultured and assayed in such a method. Furthermore, any given inhibitor can be assessed for its effect on cell motility using an assay such as this.

### Cancer staging:

A staging system is a standardized way in which clinicians describe the extent of a cancer. The most commonly used staging system for colorectal cancer is that of the American Joint Committee on Cancer (AJCC), sometimes also known as the TNM system (refer American Cancer Society for details).

The TNM system describes 3 key pieces of information: T describes how far the main (primary) tumor has grown into the wall of the intestine and whether it has grown into nearby areas; N describes the extent of spread to nearby (regional) lymph nodes. Lymph nodes are small bean-shaped collections of immune system cells that are important in fighting infections; M indicates whether the cancer has spread (metastasized) to other organs of the body.

### Colorectal cancer staging:

Colorectal cancer can spread almost anywhere in the body, but the most common sites of spread are the liver and lungs. Numbers or letters appear after T, N, and M to provide more details about each of these factors. The numbers 0 through 4 indicate increasing severity. The letter X means "cannot be assessed because the information is not available." The compounds of the present invention may be employed in the treatment of colorectal cancer at any one or more of the stages described herein.

T categories of colorectal cancer describe the extent of spread through the layers that form the wall of the colon and rectum. Tx: No description of the tumor's extent is possible because of incomplete information; Tis: The cancer is in the earliest stage (in situ). It involves only the mucosa. It has not grown beyond the muscularis mucosa (inner muscle layer); T1: The cancer has grown through the muscularis mucosa and extends into the submucosa; T2: The cancer has grown through the submucosa and extends into the muscularis propria (thick outer muscle layer); T3: The cancer has grown through the muscularis propria and into the outermost layers of the colon or rectum but not through them. It has not reached any nearby organs or tissues; T4a: The cancer has grown through the serosa (also known as the visceral peritoneum), the outermost lining of the intestines; T4b: The cancer has grown through the wall of the colon or rectum and is attached to or invades into nearby tissues or organs.

N categories indicate whether or not the cancer has spread to nearby lymph nodes and, if so, how many lymph nodes are involved. To get an accurate idea about lymph node involvement, most doctors recommend that at least 12 lymph nodes be removed during surgery and looked at under a microscope. Nx: No description of lymph node involvement is possible because of incomplete information; N0: No cancer in nearby lymph nodes; N1: Cancer cells are found in or near 1 to 3 nearby lymph nodes; N1a: Cancer cells are found in 1 nearby lymph node; N1b: Cancer cells are found in 2 to 3 nearby lymph nodes; N1c: Small deposits of cancer cells are found in areas of fat near lymph nodes, but not in the lymph nodes themselves; N2: Cancer cells are found in 4 or more nearby lymph nodes; N2a: Cancer cells are found in 4 to 6 nearby lymph nodes; N2b: Cancer cells are found in 7 or more nearby lymph nodes.

M categories indicate whether or not the cancer has spread (metastasized) to distant organs, such as the liver, lungs, or distant lymph nodes. M0: No distant spread is seen; M1a: The cancer has spread to 1 distant organ or set of distant lymph nodes; M1b: The cancer has spread to more than 1 distant organ or set of distant lymph nodes, or it has spread to distant parts of the peritoneum (the lining of the abdominal cavity).

Once a person's T, N, and M categories have been determined, usually after surgery, this information is combined in a process called stage grouping. The stage is expressed in Roman numerals from stage I (the least advanced) to stage IV (the most advanced). Some stages are subdivided with letters.

Stage 0: Tis, N0, M0: The cancer is in the earliest stage. It has not grown beyond the inner layer (mucosa) of the colon or rectum. This stage is also known as carcinoma in situ or intramucosal carcinoma.

Stage I: T1-T2, N0, M0: The cancer has grown through the muscularis mucosa into the submucosa (T1) or it may also have grown into the muscularis propria (T2). It has not spread to nearby lymph nodes or distant sites.

Stage IIA: T3, N0, M0: The cancer has grown into the outermost layers of the colon or rectum but has not gone through them (T3). It has not reached nearby organs. It has not yet spread to the nearby lymph nodes or distant sites.

Stage IIB: T4a, N0, M0: The cancer has grown through the wall of the colon or rectum but has not grown into other nearby tissues or organs (T4a). It has not yet spread to the nearby lymph nodes or distant sites.

Stage IIC: T4b, N0, M0: The cancer has grown through the wall of the colon or rectum and is attached to or has grown into other nearby tissues or organs (T4b). It has not yet spread to the nearby lymph nodes or distant sites.

Stage IIIA: One of the following applies: T1-T2, N1, M0: The cancer has grown through the mucosa into the submucosa (T1) and it may also have grown into the muscularis propria (T2). It has spread to 1 to 3 nearby lymph nodes (N1a/N1b) or into areas of fat near the lymph nodes but not the nodes themselves (N1c). It has not spread to distant sites; or T1, N2a, M0: The cancer has grown through the mucosa into the submucosa (T1). It has spread to 4 to 6 nearby lymph nodes (N2a). It has not spread to distant sites.

Stage IIIB: One of the following applies: T3-T4a, N1, M0: The cancer has grown into the outermost layers of the colon or rectum (T3) or through the visceral peritoneum (T4a) but has not reached nearby organs. It has spread to 1 to 3 nearby lymph nodes (N1a/N1b) or into areas of fat near the lymph nodes but not the nodes themselves (N1c). It has not spread to distant sites; T2-T3, N2a, M0: The cancer has grown into the muscularis propria (T2) or into the outermost layers of the colon or rectum (T3). It has spread to 4 to 6 nearby lymph nodes (N2a). It has not spread to distant sites; or T1-T2, N2b, M0: The cancer has grown through the mucosa into the submucosa (T1) or it may also have grown into the muscularis propria (T2). It has spread to 7 or more nearby lymph nodes (N2b). It has not spread to distant sites.

Stage IIIC: One of the following applies: T4a, N2a, M0: The cancer has grown through the wall of the colon or rectum (including the visceral peritoneum) but has not reached nearby organs (T4a). It has spread to 4 to 6 nearby lymph nodes (N2a). It has not spread to distant sites; T3-T4a, N2b, M0: The cancer has grown into the outermost layers of the colon or rectum (T3) or through the visceral peritoneum (T4a) but has not reached nearby organs. It has spread to 7 or more nearby lymph nodes (N2b). It has not spread to distant sites; or T4b, N1-N2, M0: The cancer has grown through the wall of the colon or rectum and is attached to or has grown into other nearby tissues or organs (T4b). It has spread to at least one nearby lymph node or into areas of fat near the lymph nodes (N1 or N2). It has not spread to distant sites.

Stage IVA: Any T, Any N, M1a: The cancer may or may not have grown through the wall of the colon or rectum, and it may or may not have spread to nearby lymph nodes. It has spread to 1 distant organ (such as the liver or lung) or set of lymph nodes (M1a).

Stage IVB: Any T, Any N, M1b: The cancer may or may not have grown through the wall of the colon or rectum, and it may or may not have spread to nearby lymph nodes. It has spread to more than 1 distant organ (such as the liver or lung) or set of lymph nodes, or it has spread to distant parts of the peritoneum (the lining of the abdominal cavity) (M1b).

### Treatment:

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a particular disease or symptom, or may be therapeutic in view of partially or completely curing a disease and/or adverse effect of the disease.

In the present invention, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

In particular, the treatment described herein relates to either reducing or inhibiting tumor growth via reducing or inhibiting proliferation of cancerous cells, or reducing the metastatic properties of cancerous cells. The prophylactic therapy as described herein is intended to encompass prevention or reduction of risk of developing metastatic cancer, due to a reduced likelihood of cancerous cells to metastasize after treatment with the compounds described herein.

### Compositions and modes of treatment:

The present invention relates further to pharmaceutically acceptable salts of the compounds described herein. The term "pharmaceutically acceptable salt" refers to salts or esters of the compounds described herein prepared by conventional means that include basic salts of inorganic and organic acids. "Pharmaceutically acceptable salts" are also inclusive of the free acid, base, and zwitterionic forms. Descriptions of suitable pharmaceutically acceptable salts can be found in Handbook of Pharmaceutical Salts, Properties, Selection and Use, Wiley VCH (2002). For therapeutic use, salts of the compounds are those wherein the counter-ion is pharmaceutically acceptable. However, salts of acids and bases which are non-pharmaceutically acceptable may also find use, for example, in the preparation or purification of a pharmaceutically acceptable compound.

Another aspect of the disclosure includes pharmaceutical compositions prepared for administration to a subject and which include a therapeutically effective amount of one or more of the compounds disclosed herein. In certain embodiments, the pharmaceutical compositions are useful for treating medical conditions associated with associated with a modified intracellular signal transduction process mediated by a proline-rich helix structure such as an infectious disease, immune disease, neurological disease, cardiometabolic disease and a cancerous disease.

The therapeutically effective amount of a disclosed compound will depend on the route of administration, the species of subject and the physical characteristics of the subject being treated. Specific factors that can be taken into account include disease severity and stage, weight, diet and concurrent medications. The relationship of these factors to determining a therapeutically effective amount of the disclosed compounds is understood by those of skill in the art.

Pharmaceutical compositions for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as antimicrobial agents, anti-inflammatory agents, anaesthetics, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

This preparation is preferably applied orally, subcutaneously, intravenously, intramuscularly, intraperitoneally and/or topically. The pharmaceutical agent comprising the compounds according to the invention is preferably used in total amounts from 0.05 to 500 mg per kg, preferably from 5 to 100 mg per kg of body weight, per 24 hours, such as 5, 10, 15, 20, 25, 30, 35, 40, 45, 50, 55, 60, 65, 70, 75, 80, 85, 90, 95 or 100 mg per kg of body weight, per 24 h.

The compositions of the disclosure can alternatively contain a pharmaceutically acceptable carrier for example substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate. For solid compositions, conventional nontoxic pharmaceutically acceptable vehicles can be used which include, for example, pharmaceutical grades of mannitol, lactose, starch, magnesium stearate, sodium saccharin, talcum, cellulose, glucose, sucrose, magnesium carbonate, and the like.

In one embodiment the invention comprise the topical and/or local administration of a compound as described herein and/or a composition comprising a compound as described here-in to a subject. The term "topical administration" refers to the delivery of a pharmacologically active agent to the skin or mucosa of a patient. Topical administration can provide a local rather than a systemic effect. The terms "topical administration" and "transdermal administration" are used interchangeably to mean administration of a pharmacologically active agent to the skin or mucosa of a patient to achieve a therapeutic effect in treating or preventing a medical disorder of the invention or discomfort at the site of topical or transdermal administration. Preferred administration modes relate to a topical solution, lotion, shake lotion, cream, ointment, gel, foam, trans-dermal patch, powder, solid form, sponge, tape, paste or tincture. Preferred embodiments relate to creams, foams, gels, lotions, and ointments.

Various additives, known to those skilled in the art, may be included in topical compositions of the present disclosure. For example, solvents, including relatively small amounts of alcohol, may be used to solubilize a compound of the invention. Other optional additives include antioxidants, fragrances, colorant, gelling agents, emulsifiers, thickening agents, stabilizers, surfactants, buffers, cooling agents (e.g., menthol) and the like. Other agents may also be added, such as antimicrobial agents, to prevent spoilage upon storage, i.e., to inhibit growth of microbes such as yeasts and molds. Examples of suitable antimicrobial agents include methyl and propyl esters of p-hydroxybenzoic acid (i.e., methyl and propyl paraben), sodium benzoate, sorbic acid, imidurea, and the like. When applied to skin, a topical composition of the present disclosure can be covered with an occlusive or non-occlusive dressing, which may be porous or non-porous, so as to protect the composition from mechanical removal during the period of treatment, e.g. a plastic film food wrap or other non-absorbent film. Various inert coverings may be employed. Non-woven or woven coverings may be employed, particularly elastomeric coverings, which allow for heat and vapor transport. These coverings can allow for cooling of the diseased site, which can provide for greater comfort, while protecting the composition from mechanical removal.

In accordance with the various treatment methods of the disclosure, the compound can be delivered to a subject in a manner consistent with conventional methodologies associated with management of the disorder for which treatment or prevention is sought. In accordance with the disclosure herein, a prophylactically or therapeutically effective amount of the compound and/or other biologically active agent is administered to a subject in need of such treatment for a time and under conditions sufficient to prevent, inhibit, and/or ameliorate a selected disease or condition or one or more symptom(s) thereof.

"Administration of" and "administering a" compound should be understood to mean providing a compound, a prodrug of a compound, or a pharmaceutical composition as described herein. The compound or composition can be administered by another person to the subject (e.g., intravenously, gel, cream, spray) or it can be self-administered by the subject (e.g., tablets, gel, cream, spray).

Dosage can be varied by the attending clinician to maintain a desired concentration at a target site (for example, the lungs or systemic circulation). Higher or lower concentrations can be selected based on the mode of delivery, for example, trans-epidermal, rectal, oral, pulmonary, or intranasal delivery versus intravenous or subcutaneous delivery. Dosage can also be adjusted based on the release rate of the administered formulation, for example, of an intrapulmonary spray versus powder, sustained release oral versus injected particulate or transdermal delivery formulations, and so forth.

The term "subject" includes both human and veterinary subjects. The term "treatment" refers to a therapeutic intervention that ameliorates a sign or symptom of a disease or pathological condition after it has begun to develop.

As used herein, the term "ameliorating", with reference to a disease or pathological condition, refers to any given beneficial effect of the treatment. The beneficial effect can be evidenced, for example, by a delayed onset of clinical symptoms of the disease in a subject, a reduction in severity of some or all clinical symptoms of the disease, a slower progression of the disease, an improvement in the overall health or well-being of the subject, or by other parameters well known in the art that are specific to the particular disease.

The present invention encompasses both therapeutic treatment and prophylactic treatment of a subject. A "prophylactic" treatment is a treatment administered to a subject, who does not exhibit signs of the medical condition or who preferably exhibits indications of developing or developing further any given medical condition, for the purpose of decreasing the risk of developing pathology or clinical symptoms. A prophylactic administration may comprise the administration of the compounds in advance of developing symptoms, thereby avoiding or reducing the subsequent occurrence of a disease. The present invention also relates to a method of treatment of subjects suffering from the various medical conditions disclosed herein. The method of treatment com-prises preferably the administration of a therapeutically effective amount of a compound dis-closed herein to a subject in need thereof.

A "therapeutically effective amount" refers to a quantity of a specified agent sufficient to achieve a desired effect in a subject being treated with that agent. For example, this may be the amount of a compound disclosed herein useful in alleviating the symptoms of one or more of the medical conditions described herein in a subject. The therapeutically effective amount or diagnostically effective amount of an agent will be dependent on the subject being treated, the severity of ill-ness, and the manner of administration of the therapeutic composition. Dosage regimens can be adjusted to provide an optimum prophylactic or therapeutic response. A therapeutically effective amount is also one in which any toxic or detrimental side effects of the compound and/or other biologically active agent is outweighed in clinical terms by therapeutically beneficial effects. A non-limiting range for a therapeutically effective amount of a compound and/or other biologically active agent within the methods and formulations of the disclosure is about 0.001 mg/kg body weight to 50 mg/kg body weight, 0.01 mg/kg body weight to about 20 mg/kg body weight, such as about 0.05 mg kg to about 5 mg/kg body weight, or about 0.2 mg/kg to about 2 mg/kg body weight.

The instant disclosure also includes kits, packages and multi-container units containing the here-in described pharmaceutical compositions, active ingredients, and/or means for administering the same for use in the prevention and treatment of diseases and other conditions in mammalian subjects.

### FIGURES

The invention is demonstrated by way of the example through the figures disclosed herein. The figures provided represent particular, non-limiting embodiments and are not intended to limit the scope of the invention.

### Brief description of the figures:

**Figure 1****:** Identification of novel small molecule transcriptional inhibitors for S100A4.
**Figure 2****:** Efficacy of S100A4 inhibition in relation to the toxicity of the selected compounds.
**Figure 3****:** Effect of the compounds E2, E10 and E12 on HCT116 cell wound healing capacity.
**Figure 4****:** Inhibitory effect of analogues A1, A2 and A3 on S100A4 mRNA inhibition.
**Figure 5****:** S100A4 mRNA expression levels and cell viability after 24 h analogue treatment of HCT116 cells.
**Figure 6****:** Inhibition of the S100A4 mRNA, protein and Wnt-signaling target gene expression after treatment with E12.
**Figure 7****:** Effect of Compound E12 on migration and proliferation capacity of the HCT116 and SW620 cell lines over 72 h.
**Figure 8****:** Maximum Tolerable Dose (MTD) evaluation of E12 in mice.
**Figure 9****:** CDX metastasis mouse model for evaluation of the capability of E12 to reduce the metastatic phenotype of HCT116/pCMV-Luc CRC cells.

### Detailed description of the figures:

**Figure 1****: Identification of novel small molecule transcriptional inhibitors for S100A4.** (A) Scheme of HCT116-pS100A4-luciferase reporter construct. Luciferase gene is driven by the human S100A4 promoter (pS100A4). (B) Scheme of the steps for the HTS conducted.

**Figure 2****: Efficacy of S100A4 inhibition in relation to the toxicity of the selected compounds.(A)** Effect of E2, E10 and E12 (A-i, A-ii and A-iii, respectively) on the S100A4 expression in HCT116 cells after 24 h of treatment with a broad range of concentrations from 1-15 µM for E2 and 0.1-15 µM for E10 and E12. S100A4 mRNA expression is relativized to DMSO control (DMSO = 1). DMSO (white bar) represents negative control for untreated cells. Each diagram shows means and error bars (95% confidence intervals) calculated from 3 independent experiments. Statistical significance was performed with ordinary one-way ANOVA using GraphPad prism 10.0 and multiple comparison was done by Dunnett's post-tests (* = p < 0.05; ** = p < 0.01; *** = p < 0.001; **** = p < 0.0001). S100A4 protein expression was determined by Western blot under the same treatment conditions with ß-actin as a protein loading control. (B) Therapeutic margin and TI for compounds E2, E10 and E12, (B-i, B-ii and B-iii, respectively) represented via displaying the compounds' S100A4 inhibition and toxicity with their calculation based on the data generated for HCT116 cells after 24 h of treatment (qPCR and MTT assay). The grey area denotes compound concentrations resulting in S100A4 inhibition below 50%, the green area indicates the therapeutic margin between the EC₅₀ and IC₅₀, and the red area denotes compound concentrations leading to a reduction in viability of more than 50%. The means and error bars representing 95% confidence intervals from at least two independent experiments.

**Figure 3****: Effect of the compounds E2, E10 and E12 on HCT116 cell wound healing capacity. (A)** Wound confluence in % monitored over 72 h using the IncuCyte Live cell imaging system. All compounds were tested from 0.1 - 15 µM. E2 displayed with concentrations from 4 - 15 µM, E10 with concentrations from 0.5 - 4 µM and E12 with concentrations from 0.5 - 6 µM. Mean values were calculated from 3 independent experiments. Panels are shown for E2, E10 and E12 (A-i, A-ii and A-iii, respectively) **(B)** Microphotographs of the wound confluence of HCT116 cells 72 h after treatment with concentrations ranging from 6 - 10 µM for E2 and 0.75 - 2 µM for E10 and E12. Results are shown as mean ± SEM of three independent experiments, performed in triplicates. Panels are shown for E2, E10 and E12 (B-i, B-ii and B-iii, respectively)

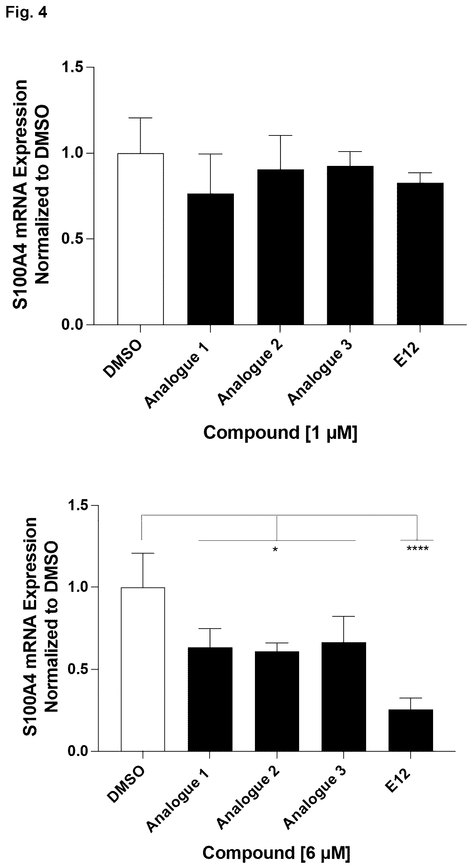

**Figure 4****: Inhibitory effect of analogues A1, A2 and A3 on S100A4 mRNA inhibition.** S100A4 mRNA Expression after 24 h of treatment at 1 µM (left) and 6 µM (right) compared to DMSO and E12 control. DMSO (white bar) represents negative control for untreated cells. E12, which represents the reference control, and the experimental conditions are depicted in black. Each diagram shows means and error bars (95% confidence intervals) calculated from 3 independent experiments. Statistical significance was performed with one-way ANOVA and multiple comparison was done by Dunnett's post-tests using GraphPad prism 10.0 (* = p < 0.05; ** = p < 0.01; *** = p < 0.001; **** = p < 0.0001).

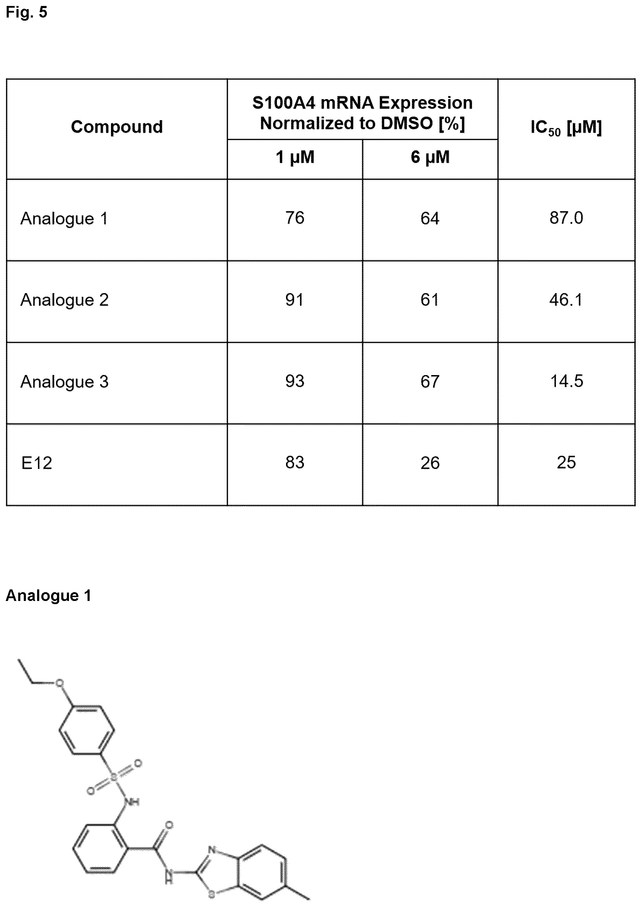

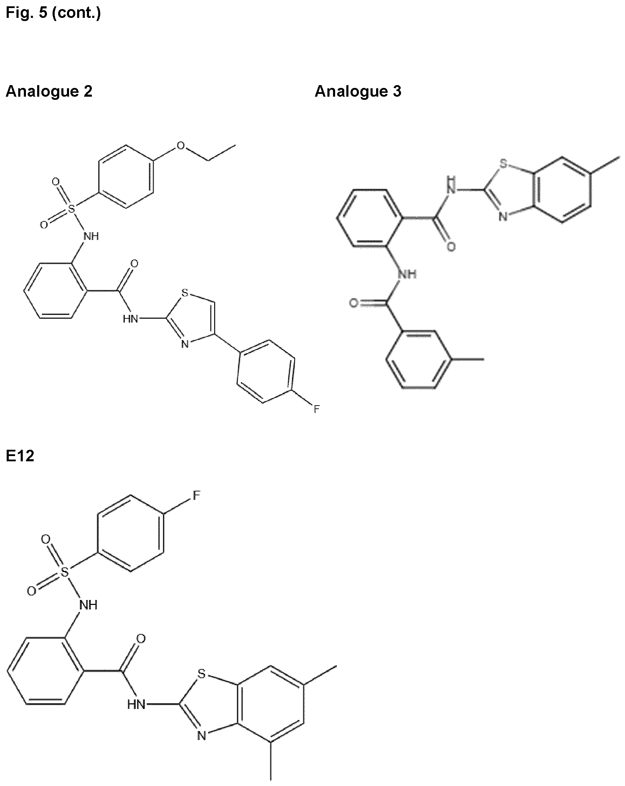

**Figure 5****: S100A4 mRNA expression levels and cell viability after 24 h analogue treatment of HCT116 cells.** S100A4 mRNA expression normalized to DMSO is represented in [%] after treatment with 1 or 6 µM of each compound. Cell viability is shown as IC₅₀ in µM and was determined after 24 h of compound treatment.

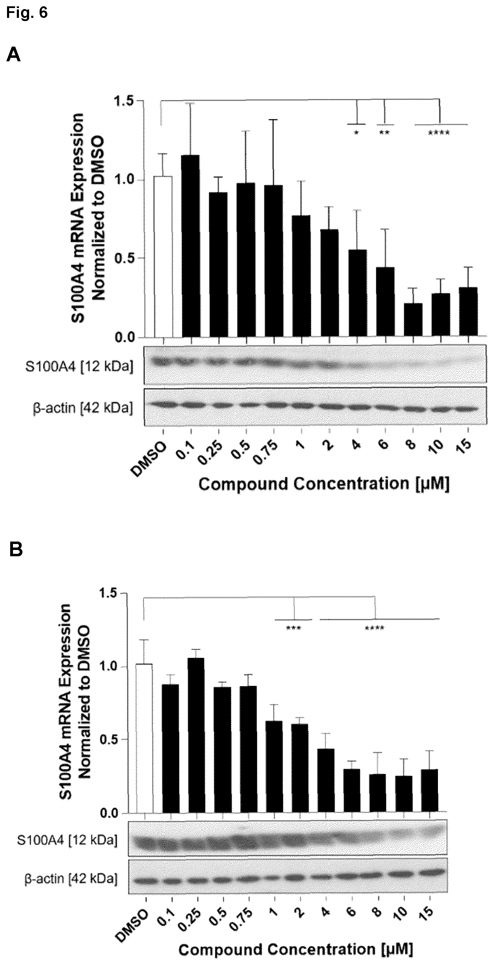

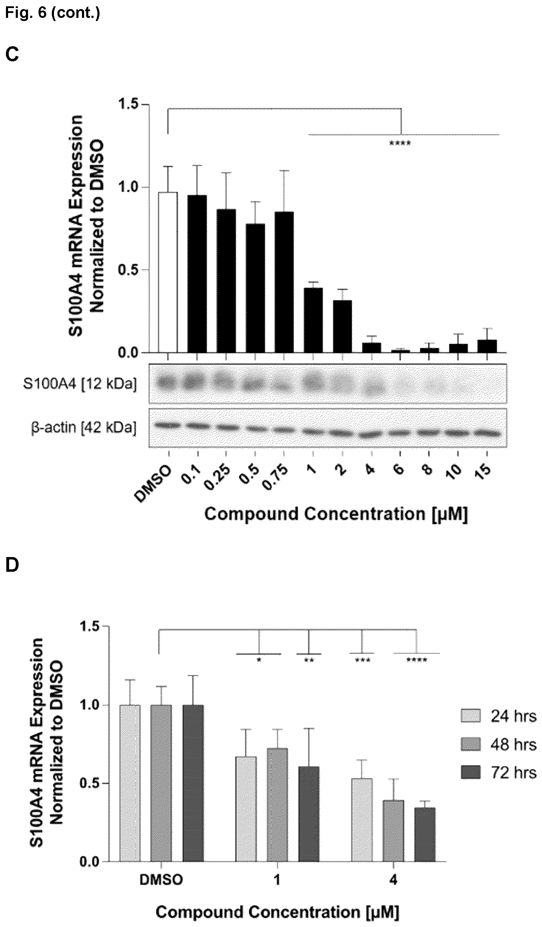

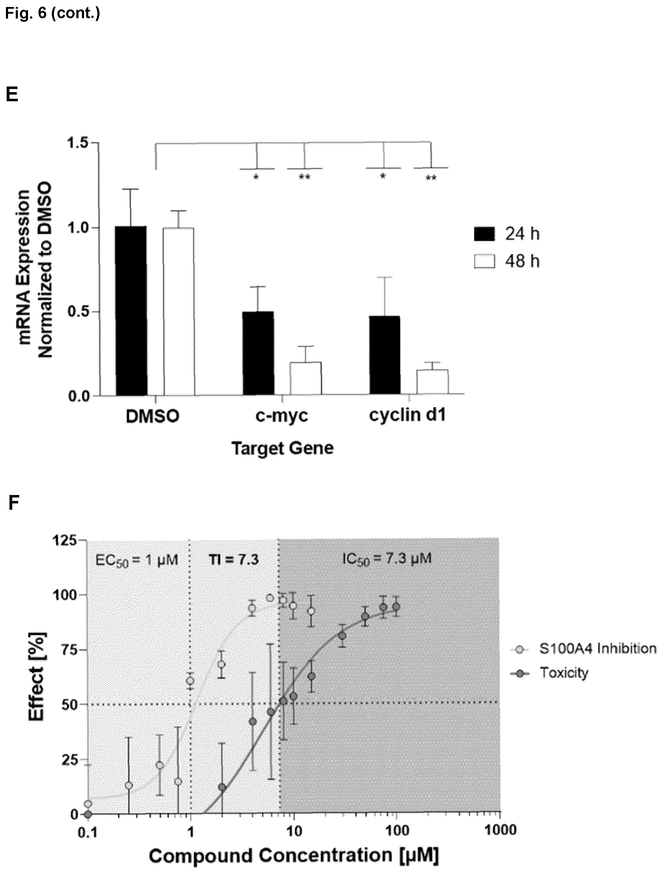

**Figure 6****: Inhibition of the S100A4 mRNA, protein and Wnt-signaling target gene expression after treatment with E12. (A)** S100A4 mRNA expression after 24 h of treatment with concentrations ranging from 0.1 - 15 µM in SW620 cells, **(B)** S100A4-mRNA expression after 48 h of treatment with concentrations ranging from 0.1 - 15 µM in SW620 cells, **(C)** S100A4-mRNA expression after 48 h of treatment with concentrations ranging from 0.1 - 15 µM in HCT116 cells, **(D)** S100A4-mRNA expression after serial time treatments in HCT116 cells at a concentration of 1 µM and 4 µM, **(E)** Effect of E12 at mRNA expression levels of Wnt-signaling associated genes c-myc and cyclin d1 after 24 h and 48 h at 6 µM, **(F)** TI for E12 after 48 h of treatment calculated by the EC₅₀ value for S100A4 mRNA inhibition and IC₅₀ value calculated by the 48 h MTT data. mRNA expression was normalized to a DMSO control (DMSO = 1). DMSO (white bar) represents negative control for untreated cells. The graph shows means and error bars (95% confidence intervals) calculated from 3 independent experiments. Statistical significance was performed using ordinary one-way ANOVA for (B-D), two-way ANOVA for (A, E) and multiple comparison was done by Dunnett's post-tests using GraphPad prism 10.0 (* = p < 0.05; ** = p < 0.01; *** = p < 0.001; **** = p < 0.0001).

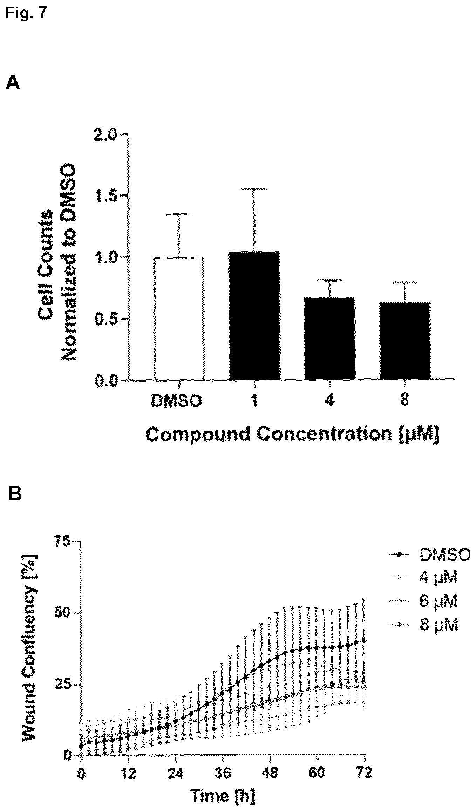

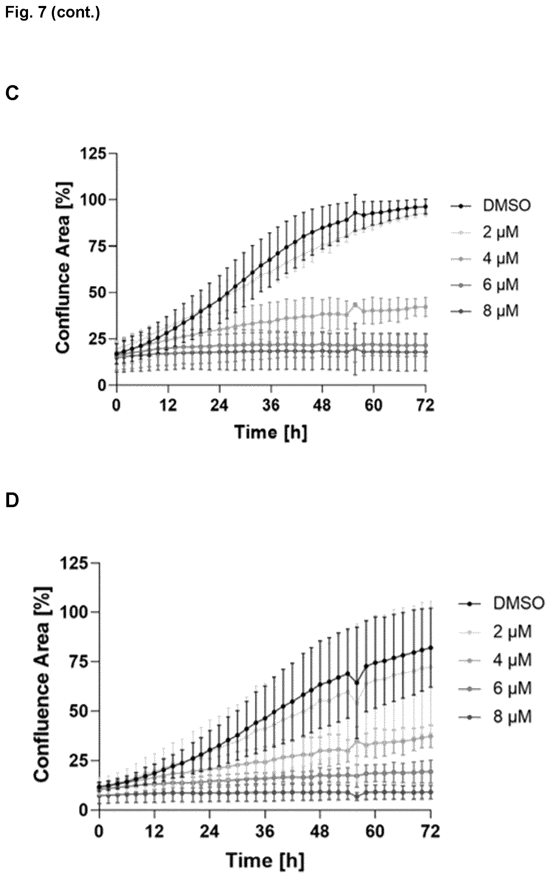

**Figure 7****: Effect of Compound E12 on migration and proliferation capacity of the HCT116 and SW620 cell lines over 72 h.** Wound healing assay observes differences in wound confluency (%) and proliferation is measured in cell confluency (%) both using the IncuCyte Live Cell Imaging system **(A)** Effect of E12 on migration capacity measured by Boyden chamber assay in HCT116 cells for concentrations 1 - 8 µM **(B)** Wound confluence after 72 h of treatment with E12 in SW620 cell line with concentrations ranging from 4 - 8 µM (C) HCT116 cells and **(D)** SW620 cells showed reduction in proliferation following 72 h treatment with E12 ranging from 2 - 8 µM. Results are shown as mean ± SEM of three independent experiments, performed in triplicates.

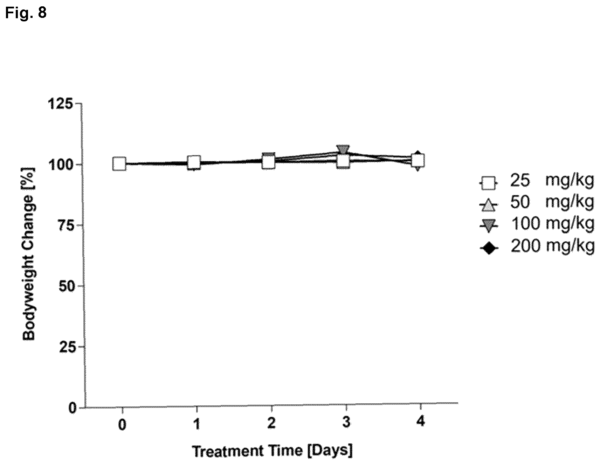

**Figure 8****: Maximum Tolerable Dose (MTD) evaluation of E12 in mice.** The mean change in mice bodyweight following daily treatment over 4 days at ranging concentrations of 25, 50, 100 and 200 mg/kg a day normalized to the bodyweight of the mice on day 0 (100%). The significance threshold was a 10% change in body weight over the 4 days.

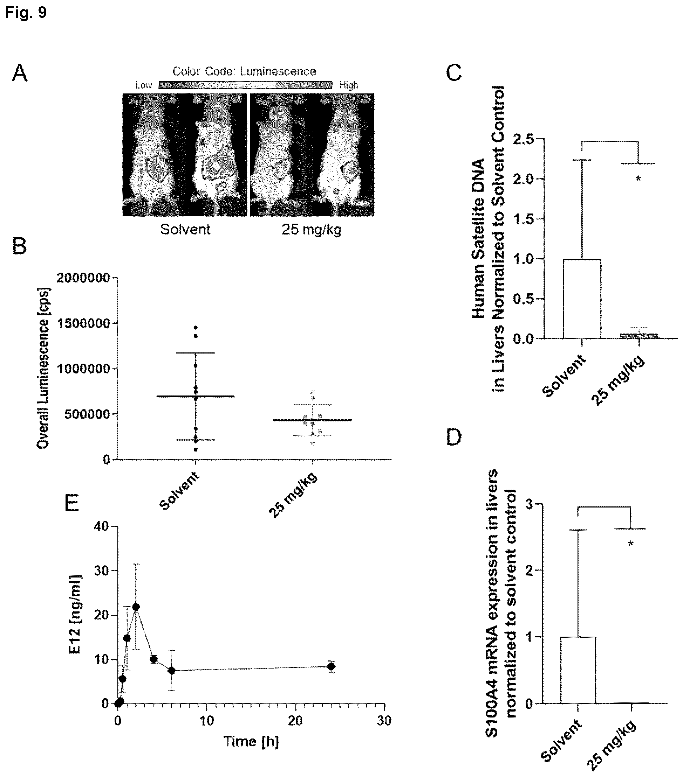

**Figure 9****: CDX metastasis mouse model for evaluation of the capability of E12 to reduce the metastatic phenotype of HCT116/pCMV-Luc CRC cells. (A)** In vivo imaging of the mice after 21 days of treatment. **(B)** Quantification of the overall luminescence signal of mice plotted as bar graphs. **(C)** Human satellite DNA of the liver of the mice after 25 days of treatment. **(D)** S100A4 expression levels of the liver of the mice after 25 days of treatment. **(E)** Plasma levels of E12 of the 25 mg/kg treated mice over 24 h. The graphs show means and error bars (95% confidence intervals) calculated from 3 independent experiments for the bar graphs and 2 independent samples for the evaluation of the plasma levels. Statistical significance was performed with ordinary one-way ANOVA and multiple comparison was done by Dunnett's post tests using GraphPad prism 8.0.2 (* = p < 0.05; ** = p < 0.01; *** = p < 0.001; **** = p < 0.0001).

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

### Methods

Compounds and treatments: The compounds identified in the HTS and used for further experiments were received from Enamine. All compounds were purchased as powder and then solubilized in DMSO (Carl Roth, Karlsruhe) to a 10 mM stock solution. Storage took place at -20°C. Each experiment was performed with DMSO as the negative control. The DMSO concentration in all wells was adjusted to the highest compound concentration which bears the highest DMSO concentration.

### Evaluation of inventive compounds

Cell Lines and cultivation: Human CRC cell lines HCT116 and SW620, initially purchased from American Type Culture Collection were cultivated in Dulbecco's Modified Eagle's Medium (DMEM; Thermo Fisher Scientific Inc, Waltham) supplemented with 10% fetal bovine serum (vol/vol) (FBS; Capricorn Scientific, Ebersdorfergrund) in a humidified incubator at 37°C with 5% CO2. The cells were regularly tested for mycoplasma contamination with MycoAlert mycoplasma detection kit (Lonza, Basel). For identification of transcriptional inhibitors of S100A4, HCT116/S100A4p-Luc cells were generated, expressing the human S100A4 promoter-driven (-1487 to +33 bp upstream of the S100A4 transcriptional start site) luciferase reporter gene [14]. Luciferase expression was regularly controlled by the Steady-Glo Luciferase Assay System (Promega, Madison).

High-throughput screening (HTS): One of the largest academic libraries with diverse small and drug like compounds in Germany was used for a screening campaign. For this screen, compounds, dissolved in dimethyl sulfoxide (DMSO) were diluted with water and prepared in 384 well white plates (CulturePlates, Revvity, Waltham) and stored at -20°C until the experiment started. HCT116/pS100A4-Luc were seeded into these 384-well plates (2x10³ cells/well; 50 µL DMEM without phenol red). This resulted into a final compound concentration of 10 µM. After 72 h of incubation, the cells were lysed, and the luciferase expression was determined by Britelite Plus Reporter Gene Assay System (Revvity, Waltham). The luciferase signal was normalized to the solvent control per plate for each compound. The most active (strongest S100A4 promoter inhibition) representative compounds of 15 hit families were selected for an investigation of the EC₅₀ (luciferase signal) and IC₅₀ (cell viability, ATPLite 1step kit, Revvity, Waltham) analysis under the same experimental conditions for 24 h, 48 h and 72 h treatments. The selected compounds were freshly bought from Enamine. The effect of these compounds was investigated in two independent batches, each performed in triplicates.

Quantitative reverse transcription - polymerase chain reaction (qRT-PCR): For mRNA-expression analysis, cells were seeded into 24-well plates (1 × 10⁵ cells/well; 500 µL DMEM). Following a 24 h incubation period, the cell medium was aspirated, and 500 µL of compound solution was added to each well. Following the varying treatment durations, total RNA was extracted using the Universal RNA Kit (Roboklon, Berlin) and quantified using a Nanodrop (Peqlab, Erlangen). The reverse transcription of 25 ng RNA into cDNA was done using the cDNA Synthesis Kit (Biozym), according to the manufacturer's protocols in volumes of 10 µL. All cDNA was stored at 4°C for immediate or at -20°C for later use. cDNA was diluted 1:3 with water, quantitative real-time polymerase chain reaction (qRT-PCR) was performed using 2 µl of cDNA with the Blue S'Green qPCR Mix (Biozym, Hessisch Oldendorf). Amplification reaction occurred in a PCR 96-well TW-MT-plate using the LightCycler 480 II (Roche, Basel), according to the manufacturer's recommendations in a volume of 10 µl. Data analyses were performed with the software of LightCycler 480 SW 1.5.1.62 (Roche, Basel). All samples were run in technical triplicates and the corresponding mean values were calculated. Each mean value of the cDNA was normalized to the respective mean value of the DMSO control.

Western blotting: For protein expression analysis, cells were seeded into 6-well plates (5 × 105 cells/well; 2 mL DMEM/well). Following a 24 h incubation period, the cell media was aspirated, and 2 mL of the prepared compound solution was added to each well. After the respective treatment durations, the cells were washed with PBS, detached via scraping and pelleted at 1000 × g for 5 min. Lysis of the cell pellet followed by dissolving them in 50 µL of RIPA buffer (composed of 50 mM Tris-HCl [pH 7.5], 150 mM NaCl, 1% Nonidet P-40, supplemented with complete protease inhibitor tablets (Roche, Basel) and incubating them on ice for 30 min, whilst vortexing the samples every 10 min. Lysates were centrifuged at 14.800 × g for 30 min at 4°C, after which the protein-containing supernatant was stored at -20°C. Protein concentrations were determined via the Pierce TM BCA Protein Assay Kit (Thermo Fisher Scientific Inc., Waltham) in order to prepare SDS loading samples containing 10 ng of protein, 100 mM DTT, 1 × NuPage^{™} LDS sample Buffer (Thermo Fisher Scientific Inc, Waltham), and PBS to a total volume of 10 µL. All samples were heated for 10 min at 95°C before loading into the SDS gels (acrylamide content: separation gel 15%, stacking gel 5%), stacking at 70 V for 30 min and separation at 120 V for 90 min. A broad range protein ladder (Spectra Multicolor, Thermo Fisher Scientific Inc., Waltham) was used to indicate the molecular weight of the proteins. The transfer was conducted with a methanol-activated PVDF membrane (Bio-Rad, Hercules) via semi-dry electro blotting employing Bio-Rad's blotting system (25 V and 1.3 A were applied for a duration of 7 min). PVDF membranes were blocked for 1 h with a TBS-T solution with 5% BSA. Membranes were cut into sections containing the S100A4 and ß-actin bands and incubated separately with their respective antibody solutions (rabbit anti-human S100A4 antibody (1:3000; Dako, Glostrup), mouse anti-human ß-actin antibody (1:50.000; Sigma-Aldrich, St. Louis); diluted in TBS-T with 5% BSA) overnight on a shaker at 4°C. The next day, membranes were washed 8 times for 5 min with TBS-T and then incubated with HRP-conjugated secondary antibodies (for the S100A4 section: goat anti-rabbit IgG anti-body (1:10.000; Promega, Madison), for the ß-actin section: goat anti-mouse IgG antibody (1:20.000; Invitrogen, Waltham) for 1 h at room temperature. Following 12 washing steps, each for 5 min with TBS-T, the Western Bright ECL substrate (Biozym, Hessisch Oldendorf) was added to the membranes. Subsequently Xray films (Kisker Biotech, Steinfurt) were exposed to the membranes. All Western blots were repeated independently three times for each compound.

MTT assay: To evaluate the effect of the compounds on the cell viability, a 3-(4,5-dimethylthiazol-2-yl)-2,5-diphenyltetrazolium bromide (MTT) assay was conducted, investigating cellular metabolic activity. Therefore, cells were seeded in 96-well plates (1 × 10⁴ cells/well; 100 µL DMEM). Following a 24 h incubation period, the cells were treated by adding 100 µL of initially double-concentrated compound solutions in DMEM. After the respective treatment durations, 20 µL of MTT (Carl Roth, Karlsruhe), dissolved in PBS and sterile filtrated, was added to each well in a final concentration of 0.5 mg/ml. Following a 1 h incubation period at 37°C, the entire media was carefully aspirated, and formazan crystals were solubilized via incubation with 100 µL DMSO for 10 min at room temperature. Quantification of metabolized MTT was executed through colorimetric measurement of absorbance at 560 nm, employing the TECAN^{™} Fluor Spectra Plus Plate Reader and the internal TECAN^{™} software (Magellan V 7.0, Tecan, Männedorf). All measured values were normalized to the sample with the lowest compound concentration. All MTT assays were repeated independently at least three times for each compound.

Wound healing assay: To investigate the migratory capacity of CRC cells in vitro, a wound healing assay was performed by first seeding a monolayer of cells (1x105 cells/well; 100 µL DMEM) in IncuCyte ImageLock 96-well plate (Essen Bioscience, Ann Arbor). Following a 6 h incubation, the cells were treated by adding 100 µL of initially double-concentrated compound solution. Subsequently, a wound width of 700 - 800 µM was created in the cellular mono-layer via the IncuCyte 96-well WoundMaker Tool (Essen Bioscience, Ann Arbor). Wound closure was observed every 2 h for 72 h using the IncuCyte Live cell imaging system (Es-sen Bioscience, Ann Arbor), capturing microscopic bright-field images of each well. The IncuCyte Zoom software enabled automated quantification of wound confluence in per-centage. The wound healing assay was repeated independently three times for each com-pound.

Proliferation assay: For the investigation of cell proliferation under compound treatment, cells were seeded in 96-well plates (1 × 10⁴ cells/well; 100 µL DMEM/well). Following a 24 h incubation period, the cells were treated by adding 100 µL of initially double-concentrated compound solutions. Proliferation was monitored over 72 h using the IncuCyte Live cell imaging system (Essen Bioscience, Ann Arbor), which determined the confluence area by analyzing captured microscopic bright-field images of each well. The IncuCyte Zoom software enabled automated quantification of confluence area in percentage. The proliferation assay was repeated independently three times.

Boyden-chamber transwell migration assay with flow cytometry: On day 0, 5 × 10⁵ cells were seeded in a 10 mL petri dish with DMEM (10% FBS). Following 24 h seeding time, the cells were starved with 0% FBS DMEM on day 1 and incubated overnight. On day 2, 235 µL of 10% FBS DMEM media was added to the bottom chamber of the migration plate, followed by addition of 100 µL of a 0.5% FBS DMEM compound/cell master mix to the upper chamber. The plate was incubated at 37°C for 18 h. On day 3, the bottom chamber media was transferred to a 96-well U-plate. 100 µL of trypsin was added to the bottom chamber for total removal of any adherent migrated cells from the plate and the membrane; the media containing cells was transferred to a second 96-well plate (U-bottom) for further flow cytometry analysis. 96-well U-plates were spun down at 300 × g for 7 min. The supernatant was removed, with the remaining U-well liquid being added to the residual pellet and the spin-down step being repeated. Following the second centrifugation step, the cells were washed in 200 µL fluorescence-activated cell sorting buffer and spun down again at the same conditions. The cells were stained with 1:1000 dilution of 4',6-diamidino-2-phenylindole (DAPI)-violet stain to FACS buffer respectively. 40 µL of the DAPI-violet dilution was added to each well. Using a multi-pipette, the samples were removed from the wells in doublets and transferred to polystyrene flow cytometry tubes using mechanical force. The BD Biosciences LSRFortessaTM machine (BD Biosciences, Franklin Lakes) was primed 3 times prior to usage. The machine was calibrated using one tube from every concentration. The computational software FlowJo v10.10 (FlowJo LLC, Ashland) was used to analyze the data. The migration assay was repeated independently three times.

Detection of human satellite DNA in mouse liver: Slices from frozen mouse liver tissues were generated with 10 µm thickness and placed into precooled tubes at -20°C. DNA was isolated after adding lysis buffer and subsequent sonication at 50% for 5 pulses using the DNA/RNA/protein extraction kit (Roboklon, Berlin). The isolated DNA was used for qPCR experiments, which were conducted as described previously. [56].

Detection of S100A4 expression in mouse liver: To detect the expression of S100A4, slices from frozen mouse liver tissues were generated with 10 µm thickness and placed into precooled tubes at - 20°C. RNA was isolated after adding lysis buffer and subsequent sonication at 50% for 5 pulses using the DNA/RNA/protein kit (Roboklon, Berlin). The isolated RNA was used to measure the expression levels in a qRT-PCR as described for the expression analysis.

In vivo tolerability for E12: A Maximum Tolerated Dose (MTD) test was carried out and was authorized by the Landesamt für Gesundheit und Soziales (LaGeSo), Berlin, Germany (Approval number E0023/23). The study was performed using 6-8 weeks old female SCID beige mice obtained from the Janvier Lab. The mice were randomly assigned to 4 groups (2 animals/group). Concentrations of 25, 50, 100, 200 mg/kg of E12 were applied orally (p.o.) daily to the mice for 5 consecutive days. The mice were observed daily, and the body weight was recorded until study termination. After study termination, animals were evaluated by gross necropsy for potential changes in the major organs.

In vivo testing of E12 in the HCT116 CRC metastasis model: A xenograft transplantation experiment was carried out and was authorized by the Landesamt für Gesundheit und Soziales (LaGeSo), Berlin, Germany (Approval number E0023/23). 6 - 8 weeks old female SCID beige mice (Charles River, Sulzfeld) were used for intrasplenic transplantation of 3 × 10⁵ HCT116/CMVp-Luc cells in anesthetized mice. The mice were assigned to 2 groups randomly (10 animals/group). For therapy, 25 mg/kg of E12 was applied orally (p.o.) daily. Control animals received solvent. The tumor growth and metastasis formation in the liver was monitored by non-invasive bioluminescence imaging (BLI, using 150 mg/kg D-luciferin in PBS) in anesthetized mice using the imaging system NightOWL LB 981 (Berthold Technologies, Bad Wildbad). The tumor growth and metastasis formation were imaged and quantified by IndiGo software version 2.0.5.0 (Berthold Technologies, Bad Wildbad, Germany). After termination of the experiment, the transplantation site organ, the spleens and the target organ of metastasis, livers, were removed, imaged, and cryopreserved in liquid nitrogen.

In vivo pharmacokinetic study: 6 - 8-week old female SCID beige mice (Charles River, Sulzfeld) were used for studying the pharmacokinetics of compound E12, which was carried out in collaboration with the EPO GmbH Berlin-Buch in compliance with the United Kingdom Coordinated Committee on Cancer Research guidelines and was authorized by the Landesamt für Gesundheit und Soziales (LaGeSo), Berlin, Germany (Approval number E0023/23). Mice were treated with a single dose of 25 mg/kg of E12 and blood was drawn at specific time points, with 2 animals per timepoint. The timepoints were 15, 30, 60 min, 2, 4, 6, and 24 h. The plasma samples were diluted 1:10 in methanol, centrifuged and the supernatant was used to measure the concentration of E12. A standard curve of E12 ranging from 0.5 to 500 ng/mL was used *for quantification.* The analysis was performed on a Vanquish Horizon HPLC coupled to a Quantiva mass spectrometer (Thermo Fisher Scientific Inc, Waltham). For injection, 1 ul of sample was used. The mobile phase used were water (buffer A) and acetonitrile (buffer B), both containing 0.1% formic acid. The proportion of buffer B changed from 45% to 100% in 4 minutes, left at 100% for other 2 minutes and reconditioning with initial conditions for other 2 minutes. Two transitions for the compound E12 were sued for detection and quantification (m/z 456 -> 179 and 456 -> 213). The analysis was performed in positive mode. Quantification was performed using the software QuantBrowser (Thermo Fisher Scientific Inc, Waltham).

Statistical analysis: Statistical analyses were performed with GraphPad Prism version 8.0.2 and 10.0 (GraphPad Software Inc, La Jolla, CA). One-way analysis of variance (ANOVA) with Dunnett's post-hoc-test was used for comparison of the control group (DMSO) with multiple compound-treated groups. In addition, Gaussian distribution was confirmed using the Kolmogorov-Smirnov test. For the calculation of the Half Maximal Inhibitory Concentration (IC₅₀), a sigmoidal dose-response - Inhibition curve fit was performed, with a baseline defining 0% viable cells determined via using a 10% DMSO positive control. This analysis therefore resulted in the calculation of an absolute IC₅₀, which was used due to widely varying maximum toxicities of the selected compounds. For the regression analysis, all compound concentrations were transformed into a logarithmic form and normalized to the control group. The Half Maximal Effective Concentration (EC₅₀) was defined by the lowest compound concentration that led to at least 50% inhibition of S100A4 mRNA expression. The IC₅₀ and EC₅₀ values were then used to calculate the Therapeutic Index (TI) as described in equation 1 $TI = {EC}_{50} / {IC}_{50}$ (1)

For the comparison of dose-response effectiveness and toxicity, S100A4 mRNA values from qRT-PCR and viability values of MTT assay were used for calculation as described in equation 2 and 3. $S 100 A 4 inhibition \left[%\right] = \left(1-S100A4 mRNA expression\right) * 100 %$ (2) $Toxicity \left[%\right] = \left(1-viability\right) * 100 %$ (3)

Dose-response curves for concentration dependent visualization of efficacy, potency and toxicity was performed using variable slope (four parameter) analysis for nonlinear regression (curve fit).

### Results

### HTS identifies novel transcriptional S100A4-inhibiting compound candidates

The HTS of more than 100,000 compounds from the screening library identified potential candidates for transcriptional inhibition of S100A4 (Fig. 1). The best hits of 15 different clusters with the strongest average inhibition of the firefly luciferase expression are referred in this work as compounds E1-E15. Detailed data are provided for three clusters, E2, E10 and E12.

### E2, E10 and E12 are the most promising novel transcriptional S100A4-inhibiting leads

To validate the inhibitory properties of the novel compounds, a comprehensive analysis was performed to assess their capabilities to inhibit S100A4 transcription. All compounds were administrated to HCT116 cells, which have a medium S100A4 expression, for 24 h and 48 h, and to SW620 cells, which have a high S100A4 expression, for 24 h at concentrations of 1 µM, 10 µM, and 30 µM, with S100A4 mRNA expression levels quantified via qRT-PCR. The summary of the data for 10 µM indicates that several compounds demonstrated limited efficacy in reducing S100A4 mRNA levels, with E1, E5 and E11 showing reductions of less than 15%, while E4, E8 and E9 even led to increased S100A4 mRNA expression after a 24 h or 48 h of treatment. In contrast, E6, E7, E10, E12 and E15, exhibited a pronounced reduction of S100A4 mRNA levels already after 24 h. Other compounds, such as E2, E3, E13 and E14, demonstrated stronger reductions only after 48 h of treatment. In the SW620 cell line, the compounds were generally less effective, with only E12 showing a strong inhibition of S100A4 mRNA expression. Given the fact that the HCT116 cell line was chosen as the primary test system for HTS, the selection of the potential leading candidates focused primarily on their performance in this cell line. The selection criteria of the three most promising compounds for further evaluation were based on two key factors: firstly, the strongest reduction of S100A4 mRNA level at 10 µM after 48 h of treatment, and secondly, a concentration- and time-dependent reduction of S100A4 mRNA expression. Through this initial re-screening of the 15 identified HTS hits, E2, E10, and E12 emerged as the most effective inhibitors of S100A4 mRNA expression. Consequently, the following experiments focused on the evaluation of these compounds.

### Selected compounds inhibit the S100A4 mRNA and protein expression

To achieve a more precise characterization of the concentration-dependent inhibition of S100A4, the selected compounds (E2, E10 E12) were further evaluated over an extended concentration range (1-15 µM for E2 and 0.1-15 µM for E10 and E12) at both the mRNA and protein level following a 24 h treatment (Fig. 2A). All 3 compounds reduce the S100A4 mRNA expression in a concentration-dependent manner. E2 showed significant inhibition at concentrations starting from 6 µM (EC₅₀ = 10 µM). At the protein level, reduction of S100A4 was only visible at 15 µM, demonstrated by Western blot. E10 and E12 exhibited higher potency and more pronounced inhibitory effects. E10 showed significant reduction in S100A4 mRNA expression at concentrations as low as 2 µM (EC₅₀ = 15 µM). However, a reduced protein expression could be seen after 24 h only at the highest concentration of 15 µM in the Western blot. By contrast, E12 reached a maximum of inhibition of 75% at 6 µM, with the lowest EC₅₀ of the selected compounds (EC₅₀ = 4 µM). S100A4 protein level analysis showed a concentration-dependent inhibition with E12 beginning at 4 µM. These findings confirm the transcriptional inhibitory effect of the selected HTS-identified compounds, as evidenced by the reduction of S100A4 mRNA expression.

### E12 exhibits the highest Therapeutic index (TI) and most favourable impact on cell viability of the selected compounds

To systematically assess the effects of the newly identified compounds on cell viability, an MTT assay was performed. The selected compounds exhibited a concentration-dependent decrease in cell viability. E2 showed a 50% reduction of the cell viability with an IC₅₀ value of 6.2 µM after 24 h of treatment. E10 displayed a similar IC₅₀ value after 24 h (IC₅₀ = 8.9 µM). Further, out of the 3 compounds, E12 exhibited the highest IC₅₀ value of 25.0 µM after 24 h, which decreased to 6.1 µM after 48 h of treatment in HCT116 cells. By correlating the compound-specific effects on the reduction of viability with their inhibition of S100A4 mRNA expression (see Selected compounds inhibit the S100A4 mRNA and protein expression), the concentration-response-curves enabled a comprehensive evaluation of the therapeutic potential of the novel compounds. This analysis also served as a basis for calculating the TI, a key parameter that represents an easy comparable factor for determining the therapeutic margin of an active compound. For the 24 h treatments in HCT116 cells with the selected compounds, E2 and E10 displayed no discernable therapeutic margin, reflected by their low TI of 0.6. E12 showed the highest TI of 6.3. At concentrations of 4 µM and higher, E12 effectively inhibited S100A4 mRNA expression (over 50%) while causing relatively minor decrease in cellular viability. Accordingly, E12 displayed the most promising TI of all 3 selected compounds.

### Compounds E2, E10 and E12 reduce wound healing capacity in HCT116 cells

Next, the selected compounds were applied to a wound healing assay, demonstrating a concentration-dependent inhibition of wound closure. Consistent with its relatively weaker inhibition of S100A4, E2 had the least distinct effects on wound healing, with a concentration of 15 µM inhibiting the wound healing by 39% after 72 h of treatment compared to the control. In contrast, E10 and E12 showed stronger inhibition of the wound healing capacity of the cells. E10 inhibited wound healing at concentrations as low as 0.5 µM, with a maximum effect observed at 2 µM, resulting in 63% inhibition of the wound healing at 72 h post-treatment. E12 showed a similar inhibition of wound healing at a concentration of 0.5 µM, with wound healing reduced to 27% confluence at 4 µM after 72 h. These findings demonstrate a clear correlation between S100A4 inhibition and the reduction of wound healing capacity, indicating that the selected compounds restrict cell migration and proliferation in a concentration-dependent manner.

### S100A4 inhibition of E12 analogues

Compound E12, N-(4,6-dimethylbenzo[d]thiazol-2-yl)-2-((4-fluorophenyl)sulfonamido)benzamide, was the most effective compound for S100A4 mRNA inhibition identified in an extensive HTS. In addition, it displayed a high TI compared to the other selected compounds which concludes that it will be the most promising candidate for in vivo studies. Chemically, the core structure consists of an anthranilic acid, which is decorated with a fluorophenyl-sulfonamide and an amino-benzothiazole group. The calculated physical-chemical properties [31] predict a high degree of drug-like properties for E12, adhering to the Lipinski rule of five [32] for orally absorbable small molecules likely to exhibit an acceptable oral bioavailability.

Considering that the tested compound E12 was derived from chemical clusters of the HTS, 3 structural analogs of E12 were selected to test for improved performance in either efficacy or cell viability. All analogues of E12 named Analogue 1 (A1), Analogue 2 (A2) and Analogue 3 (A3) (Figure 5) were tested in HCT116 cells at 1 and 6 µM over 24 h to observe their S100A4 mRNA inhibitory capacity. For A1 the fluor in the phenyl group was exchanged by an ethoxy group and the amino-benzothiazole group only harbours one methyl group. Further, for A2 the fluor was exchanged by an ethoxy group as well and the benzothiazole group is replaced by a thiazol connected to an fluorophenyl group. In A3 the amino-benzothiazole group harbours one methyl group and the sulfonamide is replaced by a carboxyl group connected to the phenyl which is decorated by one methyl group. Each experiment included a treatment of E12 at 1 µM and 6 µM as a reference control. For the 1 µM treatments, A1, A2 and A3 reduced the S100A4 mRNA expression by 24%, 10% and 8% respectively; the corresponding E12 treatment showed to reduce the mRNA expression by 18% (Figure 4). For the 6 µM treatments, A1, A2 and A3 reduced the S100A4 mRNA expression by 37%, 39% and 34% respectively; the corresponding E12 treatment showed a reduction of 74% at 6 µM (Figure 4).

### Analogues showed varying effect on cell viability compared to E12

The cellular viability of the analogues was tested to assess their toxicity compared to E12. All MTT assays were conducted in HCT116 cells over 24 h measuring viability as the IC₅₀ value. The IC₅₀ of A1 was 87.0 µM, which is 6-fold higher than that of E12 as per table 2. A2 showed to have an IC₅₀ value of 46.1 µM three-fold higher than of E12. Conversely A3 had a corresponding IC₅₀ value of 14.5 µM.

### E12 reduces S100A4 mRNA and protein expression under various conditions

The impact of E12 on S100A4 mRNA and protein levels were further evaluated over a broad concentration range (0.1-15 µM) following 24 and 48 h treatment in both HCT116 and SW620 cell lines (Figure 6A-C). E12 reduced S100A4 mRNA expression in a concentration-dependent manner in both cell lines over each period; significant inhibition at the mRNA level after 48 h started from 1 µM treatment onwards with 40% in HCT116 and 38% inhibition in SW620 cells (Figure 6B, C). The strongest inhibition observed after 48 h was at 6 µM in HCT116 (97% inhibition, Figure 6C) and 8 µM after 24 h and 10 µM after 48 h in SW620 cells (73% and 75% inhibition respectively, Figure 6A-B). A similar concentration-dependent effect was translated into the protein expression. Fading protein bands can be witnessed for SW620 cells starting from 6 µM for 24 h and from 8 µM for 48 h treatment. For the 48 h treatment duration in HCT116 cells a reduction of the protein band is visible starting from 6 µM. The next step in the transcriptional inhibition analysis of E12 was its impact on the S100A4 mRNA expression levels following serial treatment in HCT116 cells at 1 µM and 4 µM. As can be seen in Figure 6E, S100A4 expression was reduced by 32% after 24h treatments at 1 µM; upon continuous application of the compound at this concentration, the expression reduction was maintained at 27% and 39% over 48 and 72 h respectively. 4 µM treatments reduced the expression to around 50% compared to the DMSO control after 24 h, with a stepwise decrease of S100A4 expression after 48 h being reduced by 60%. This reduction was maintained at 72 h at 65% and showed that the continual application of the 1 µM and 4 µM concentration did not increase or decrease the expression and the cells were not able to rescue the S100A4 expression under continual treatment of E12. Furthermore, the TI for E12 after 48 h of treatment in HCT116 cells was calculated using metabolism based MTT 48 h assay data and the S100A4 mRNA expression results from Figure 6C. The TI is 7.3 over 48 h and therefore consistent with the 24 h TI for E12 (Figure 6F).

### E12 reduces Wnt-signaling pathway associated genes

To investigate if E12 was potentially interrupting the Wnt-signaling pathway following treatment, the impact of E12 on the mRNA expression of the two Wnt-signaling target genes c-myc and cyclin d1 was investigated in HCT116 cells over 24 and 48 h treatment at 6 µM (Figure 6E). The compound reduced c-myc expression in a time-dependent manner by 50% over 24 h and subsequently 80% over 48 h. Furthermore, cyclin d1 expression reduction followed a similar pattern at 66% reduction after 24 h and 83% reduction after 48 h.

### E12 reduces the metastatic phenotype of CRC cells in vitro

The wound healing assay was additionally carried out in the SW620 cell line to assess migration capacity reduction. Notably, the first substantial drop in wound healing ability to 26.9% of wound confluency was seen after 72 h of treatment which is a drop by 13.1% compared to the control following treatment with 4 µM. Inhibition of wound healing increased at 6 µM, which showed the strongest inhibition with 23 % of wound confluency restored after 72 h; an inhibition plateau was reached here as 8 µM showed no increased efficacy (Figure 7B). A second migration assay known as the Boyden Chamber Assay was carried out in HCT116 cells treated at 1, 2, 4 and 8 µM (Figure 7A). At 4 µM, a drop of 50% migration capacity is observed compared to the control which was further reduced by 8 µM, resulting in a 60% inhibition. Finally, a third phenotypic assay was done in HCT116 and SW620 cells known as the proliferation assay. This assay measures cell confluency from time points 0 h to 72 h following a broad range concentration treatment as shown in Figure 7 C and D respectively. E12 reduced proliferation in HCT116 cells from a concentration of 4 µM onward, reducing cell confluency by 45% and higher concentrations of 6 and 8 µM prevented any proliferation of the cells compared to the control. The SW620 (Figure 7D) cell line showed a similar pattern of proliferation inhibition following treatment: proliferation was reduced from 4 µM onward, reducing cell confluency by 40% compared to the solvent control.

### Maximum tolerable dose (MTD) testing of E12 in vivo

The maximum tolerable dose escalation trial was done over 5 days treating four dosage groups (25, 50, 100, 200 mg/kg) every 24 h. The results depicted in Figure 8 demonstrate the bodyweight change in percentage per dosage group over the 5 treatment days normalized to the bodyweight of the mice on day 0. There was no significant reduction or increase in body weight after the 4 days, of which the threshold would be defined as an increase or decrease of body weight by 10%. This indicates that E12 is tolerable for in vivo models in an acute manner and can be applied to carry out further vivo testing.

### E12 reduces the metastatic capabilites of HCT116 cells in metastasis model in vivo

After testing for the maximum tolerable dose of E12, a metastasis mouse model was applied using HCT116/pCMV-Luc cells which were intrasplenically transplanted. 20 female SCID/beige mice were assigned to 2 groups: the control group which received solvent and the 25 mg/kg group of E12. Substance and solvent were applied daily p.o. After 21 days of treatment the overall bioluminescence in the mice was quantified. It can be seen in Figure 9B that E12 reduced the overall bioluminescence of the 25 mg/kg treated mice compared to the solvent control group. The molecular analysis of human satellite DNA and the S100A4 expression in the liver of the mice after 25 days showed a significant reduction in the treatment group for both analyses compared to the solvent control (Figure 9C, D). This suggests that E12 can reduce S100A4 expression and metastasis formation in vivo. Further, a brief pharmacokinetic study was conducted measuring the amount of E12 in the blood of 2 SCID/beige mice receiving 25 mg/kg. A spike of 21.9 ng/ml is visible after 2 h in Figure 9E. The amount of E12 drops down to 8.4 ng/ml after 24 h, showing that E12 was successfully absorbed into the plasma of the mice.

### Summary of Results

The inventors identified a novel S100A4 transcriptional inhibitor E12, a [(fluorophenyl) sulfonyl] benzamide-based compound that shows high efficiency in reducing the metastatic potential of CRC cells. Thereby, this compound provides a novel efficacious option for targeted therapy for clinical applications of both pre-metastatic and metastatic patients. E12 outperformed all other drug candidates identified in the HTS on the mRNA expression level. This compound demonstrated the highest efficacy of S100A4 expression inhibition at both mRNA and protein levels, and cellular viability assays determined low toxicity of the compound. Subsequent restriction of the metastatic phenotype in vitro by E12 was demonstrated; hereby, migration and proliferation capacities of CRC cells were significantly reduced. Further assessment through in vivo studies revealed E12 to reduce the metastatic potential of the human CRC cell line HCT116 in a CDX metastasis mouse model. These described characteristics of high efficacy and potency in tandem with low toxicity primes this compound to have a favorable therapeutic margin and holds great promise for application to patients in the future.

One of the key aspects in drug discovery is identification of a compound that competently executes its function whilst remaining tolerable for the patient. In this regard, E12 proved itself as functional anti-metastatic compound that is lowly toxic to cells. This is reflected by the TI, a quantitative measurement of the compound's reduction of cell viability (IC₅₀ value) and its efficacy in reducing S100A4 mRNA expression, reflected by the EC₅₀ value. The E12 TI score of 6.3 after 24 h treatment represents the broadest therapeutic margin out of the candidates tested. Furthermore, the score of 6.1 after 48 h treatment reflects maintenance of this ratio over time, highlighting the compound's maintenance of potency. The high TI is attributed to E12 reducing S100A4 mRNA expression by 50% at concentrations as low as 4 µM after 24 h, whilst in parallel only exhibiting an IC₅₀ value of 25.0 µM. The TI is contingent upon factors such as body mass, biological sex, and other physiological variables [33], and therefore whilst the in vitro data suggests a preliminary TI, it cannot be directly inferred to a patient context. When E12 is ranked in relation to other currently clinically trialed anti-S100A4 transcription inhibitory drugs such as niclosamide (EC₅₀ of 1 µM after 24 h treatment), which possesses a IC₅₀ of 2.2 after 24 h [35], E12 shows a substantial improvement in safety to efficacy ratio. Nevertheless, this compound will need an intensive structure activity relationship study, since E12 and its 2-[(4-fluorophenyl) sulfonyl] benzamide-based structure hold promise as a lead to further increase its efficacy while simultaneously reducing its toxicity through further medicinal chemistry studies.

The transcription of S100A4 has high relevancy in promoting the execution of CRC metastasis through intracellular and extracellular mechanisms, as well as to modulate the tumor microenvironment to a pro-metastatic state. Therefore, the significant reduction of S100A4 gene expression by E12 over 24- and 48-hour treatment periods hold high potential for treating patients with a high-risk for metastasis. E12 impact on S100A4 mRNA, protein and metastatic phenotype inhibition was effective in both colon cancer cell lines HCT116 and SW620. HCT116 harbours a different genetic profile to SW620, containing a three-base deletion in exon 3 (Ser45) in CTNNB1 and a correlating WT APC gene, whilst the SW620 cell line carries mutations in the APC gene and a wild-type CTNNB1 gene [36,37]. Both mutations result in an aberrant canonical Wnt-signaling pathway, and hence increased S100A4 expression [38-41]. This dual effect indicates the compound's ability to treat different mutations that may lead to S100A4 overexpression. The ability to treat patients with high tumour heterogeneity is pivotal in metastasis therapy due to its prominent role in drug resistance. It should be noted that SW620 exhibits a more metastatic phenotype, most likely due the cell line's origin from a lymph node metastasis [42]. This result greatly supports the use of this compound to treat metastatic stage IV patients, as SW620 thoroughly reflects a metastatic cell type. Concurrently, E12's effect on the primary tumour derived HCT116 cell line indicates its application potential in patients at risk of cancer dissemination (stages I-III). This is particularly relevant when considering that 50% of all patients diagnosed in stages I-III will undergo metastasis throughout the time course of their disease [43]. Thus, it can be presumed that E12 or other compounds of the invention could be used clinically in a preventive setting to treat patients that are pre-metastatic (for prevention) and metastatic (for reduction and restriction). In addition, E12 showed to reduce mechanosensory-driven and chemotaxis-driven migration. These are both important mechanisms to reduce dissemination of cancer cells to the pre-metastatic niche. Notably, E12 reduced migration more efficiently in the wound healing assay than in the Boyden chamber assay. We hypothesize this could be due to the compound only becoming active at reducing S100A4-induced migration after 18 h of treatment; in the Boyden chamber assay the treatment is halted at 18 h. This hypothesis is based on the wound healing migration inhibition beginning 18 h post treatment and peaking at 60 h post treatment at 4 µM and 8 µM as well. Any wound healing inhibition prior to 18 h is hypothesized to be due to proliferation reduction which starts almost immediately at these concentrations as shown in the proliferation assay. We hypothesize proliferation is reduced by E12 here through general Wnt-signaling disruption. This would then result in the demonstrated cyclin d1 and c-myc expression reduction, two target genes of the Wnt-signaling pathway [44,45] that are known to drive proliferation. For context, Cyclin D1 is an allosteric master regulator of the cell cycle, more specifically it works in concert with the activating cyclin-dependent kinases (CDK4/6) to allow for G1/S checkpoint progression [46,47] and hence tumour growth. The key oncogenic transcription factor c-myc drives cancer by promoting proliferation and survival [48-50]. Reduction of expression of these two oncogenes is not only indicative of Wnt-signaling pathway interruption, but also reflective of the anti-cancerous properties of E12. Notably, this data indicates the possibility that E12 is applicable in treating not only CRC, but also in a wider range of clinical contexts such as other S100A4-prognostic cancers that display aberrant Wnt-signaling pathways, such as breast, lung, gastric, melanoma and ovarian cancer [51-55]. Furthermore, this reduction of proliferation by an anti-metastatic compound plays a crucial role to inhibit the establishment of metastasis in peripheral organs.

In vivo CDX metastasis mouse model experiments have shown that lower concentrations of E12 causes no toxic side effects in mice after 25 days of treatment, while influencing the metastasis formation as seen in the 25 mg/kg group. This reduction of overall luminescence after 21 days compared to the solvent treated control, as well as the human satellite DNA reduction in treated vs. control, can conclude E12's efficacy in vivo to reduce formation of liver metastases. The S100A4 mRNA levels in the liver were substantially lower compared to the solvent treated group, demonstrating that the reduction of metastasis is due to the inhibition of the S100A4 gene expression.

As the lead candidate identified in this HTS study, the 2-[(4-fluorophenyl) sulfonyl] benzamide-based E12 holds great promise for reducing and restricting the most lethal attribute of cancer: metastasis, and thus can be considered a potential new therapy approach amidst the ongoing anti-metastasis therapy renaissance.

### Conclusion of the Examples

The inventors show that the overexpression of S100A4 is associated with poor prognosis of affected CRC patients. The inventors also show the link of these underexplored biomarkers to Wnt-signaling. These findings prompted the inventors to exploit these targets by intervention of tumor progression and metastasis formation using repositioned small molecule inhibitors. These small molecule inhibitors showed unexpected favorable characteristics for S100A4 transcriptional inhibition while also proving viability in a mouse model. This is a strong indication, that targeting of this key pathway and molecules causally involved in cancer metastasis can lead to efficient intervention particularly for patients, who are at high risk for metastasis due to S100A4 overexpression. Thus, treatment of those patients stratified for S100A4 overexpression will be beneficial for these patients and holds enormous promise for clinical use.

### REFERENCES

[1] Cancer Today. Available online: http://gco.iarc.fr/today/home (accessed on 8 October 2024)
[2] Dillekås H, Rogers MS, Straume O. Are 90% of deaths from cancer caused by metastases?. Cancer Med. 2019;8(12):5574-5576. doi:10.1002/cam4.2474
[3] National Cancer Institute-SEER Program Colorectal Cancer-Cancer Stat Facts. [(accessed on 10 October 2024)]; Available online: https://seer.cancer.gov/statfacts/html/colorect.html]
[4] Elferink MA, de Jong KP, Klaase JM, Siemerink EJ, de Wilt JH. Metachronous metastases from colorectal cancer: a population-based study in North-East Netherlands. Int J Colorectal Dis. 2015;30(2):205-212. doi:10.1007/s00384-014-2085-6
[5] Davies BR, Davies MP, Gibbs FE, Barraclough R, Rudland PS. Induction of the metastatic phenotype by transfection of a benign rat mammary epithelial cell line with the gene for p9Ka, a rat calcium-binding protein, but not with the oncogene EJ-ras-1. Oncogene. 1993;8:999-1008
[6] Maelandsmo GM, Hovig E, Skrede M, Engebraaten O, Florenes VA, Myklebost O, Grigorian M, Lukanidin E, Scanlon KJ, Fodstad O. Reversal of the in vivo metastatic phenotype of human tumor cells by an anti-CAPL (mts1) ribozyme.Cancer Res. 1996;56:5490-5498. 79.
[7] Ambartsumian NS, Grigorian MS, Larsen IF, Karlstrom O, Sidenius N, Rygaard J,Georgiev G, Lukanidin E. Metastasis of mammary carcinomas in GRS/A hybrid micetransgenic for the mts1 gene. Oncogene. 1996;13:1621-1630.
[8] Davies MP, Rudland PS, Robertson L, Parry EW, Jolicoeur P, Barraclough R. Expression of the calcium-binding protein S100A4 (p9Ka) in MMTV-neu transgenic mice induces metastasis of mammary tumors. Oncogene. 1996;13:1631-1637
[9] Stein U, Arlt F, Walther W, Smith J, Waldman T, Harris ED, Mertins SD, Heizmann CW, Allard D, Birchmeier W, Schlag PM, Shoemaker RH. The metastasis-associated gene S100A4 is a novel target of β-catenin/T-cell factor signaling in colon cancer. Gastroenterology. 2006;131:1486-1500
[10] Khan MI, Yuan T, Chou RH, Yu C. S100A4 inhibits cell proliferation by interfering with the S100A1-RAGE V domain. PLoS One. 2019 Feb 19;14(2):e0212299. doi: 10.1371/journal.pone.0212299. PMID: 30779808; PMCID: PMC6380570.
[11] Rudland PS, Platt-Higgins A, Renshaw C, West CR, Winstanley JH, Robertson L, Barraclough R (2000) Prognostic significance of the metastasis-inducing protein S100A4 (p9Ka) in human breast cancer. Cancer Res 60: 1595-1603\
[12] Cho YG, Nam SW, Kim TY, Kim YS, Kim CJ, Park JY, Lee JH, Kim HS, Lee JW, Park CH, Song YH, Lee SH, Yoo NJ, Lee JY, Park WS (2003) Overexpression of S100A4 is closely related to the aggressiveness of gastric cancer. APMIS 111 (5): 539-545
[13] Heman R, Fasheh R, Calabrese C, Frank AJ, Maclean KH, Allard D, Barraclough R, Gilbertson RJ (2003) ERBB2 up-regulates S100A4 and several other prometastatic genes in medulloblastoma. Cancer Res 63 (1): 140-148
[14] Missiaglia E, Blaveri E, Terris B, Wang YH, Costello E, Neoptolemos JP, Crnogorac- Jurcevic T, Lemoine NR (2004) Analysis of gene expression in cancer cell lines identifies candidate markers for pancreatic tumorigenesis and metastasis. Int J Cancer 112 (1): 100-112
[15] Dahlmann M, Monks A, Harris ED, et al. Combination of Wnt/β-Catenin Targets S100A4 and DKK1 Improves Prognosis of Human Colorectal Cancer.Cancers (Basel). 2021;14(1):37. doi:10.3390/cancers14010037
[16] Treese C, Hartl K, Pötzsch M, Dahlmann M, von Winterfeld M, Berg E, Hummel M, Timm L, Rau B, Walther W, Daum S, Kobelt D, Stein U. S100A4 Is a Strong Negative Prognostic Marker and Potential Therapeutic Target in Adenocarcinoma of the Stomach and Esophagus. Cells. 2022 Mar 21;11(6):1056. doi:10.3390/cells11061056. PMID: 35326507; PMCID: PMC8947340.
[17] Zhang J, Gu Y, Liu X, Rao X, Huang G, Ouyang Y. Clinicopathological and prognostic value of S100A4 expression in non-small cell lung cancer: a meta-analysis. Biosci Rep. 2020 Jul 31;40(7):BSR20201710. doi: 10.1042/BSR20201710. PMID: 32696952; PMCID: PMC7396424.
[18] The Cancer Genome Atlas Network. Comprehensive molecular characterization of human colon and rectal cancer. Nature 487, 330-337 (2012).
[19] Dahlmann M, Okhrimenko A, Marcinkowski P, et al. RAGE mediates S100A4-induced cell motility via MAPK/ERK and hypoxia signaling and is a prognostic biomarker for human colorectal cancer metastasis. Oncotarget. 2014;5(10):3220-3233.doi:10.18632/oncotarget.1908
[20] Cerezo LA, Remáková M, Tomčik M, et al. The metastasis-associated protein S100A4 promotes the inflammatory response of mononuclear cells via the TLR4 signalling pathway in rheumatoid arthritis. Rheumatology (Oxford). 2014;53(8):1520-1526. doi:10.1093/rheumatology/keu031
[21] Kriajevska M, Tarabykina S, Bronstein I, et al. Metastasis-associated Mts1 (S100A4) protein modulates protein kinase C phosphorylation of the heavy chain of nonmuscle myosin. J Biol Chem. 1998;273(16):9852-9856. doi:10.1074/jbc.273.16.9852
[22] Dulyaninova N.G., Ruiz P.D., Gamble M.J., Backer J.M., Bresnick A.R. S100A4 regulates macrophage invasion by distinct myosin-dependent and myosin-independent mechanisms. Mol. Biol. Cell. 2018;29:632-642. doi: 10.1091/mbc.E17-07-0460.
[23] Belot N, Pochet R, Heizmann CW, Kiss R, Decaestecker C. Extracellular S100A4 stimulates the migration rate of astrocytic tumor cells by modifying the organization of their actin cytoskeleton. Biochim Biophys Acta. 2002;1600:74-83.
[24] Tochimoto M, Oguri Y, Hashimura M, et al. S100A4/non-muscle myosin II signaling regulates epithelial-mesenchymal transition and stemness in uterine carcinosarcoma. Lab Invest. 2020;100(5):682-695. doi:10.1038/s41374-019-0359-x
[25] Li ZH, Bresnick AR. The S100A4 metastasis factor regulates cellular motility via a direct interaction with myosin-IIA. Cancer Res. 2006;66(10):5173-5180. doi:10.1158/0008-5472.CAN-05-3087
[26] Bjørnland K, Winberg JO, Odegaard OT, et al. S100A4 involvement in metastasis: deregulation of matrix metalloproteinases and tissue inhibitors of matrix metalloproteinases in osteosarcoma cells transfected with an anti-S100A4 ribozyme. Cancer Res. 1999;59(18):4702-4708.
[27] Saleem M, Kweon MH, Johnson JJ, et al. S100A4 accelerates tumorigenesis and invasion of human prostate cancer through the transcriptional regulation of matrix metalloproteinase 9. Proc Natl Acad Sci U S A. 2006;103(40):14825-14830. doi:10.1073/pnas.0606747103
[28] Yonemura Y, Endou Y, Kimura K, et al. Inverse expression of S100A4 and E-cadherin is associated with metastatic potential in gastric cancer. Clin Cancer Res. 2000;6(11):4234-4242.
[29] Keirsebilck A, Bonné S, Bruyneel E, et al. E-cadherin and metastasin (mts-1/S100A4) expression levels are inversely regulated in two tumor cell families. Cancer Res. 1998;58(20):4587-4591.
[30] Burock S, Daum S, Keilholz U, et al. Phase II trial to investigate the safety and efficacy of orally applied niclosamide in patients with metachronous or synchronous metastases of a colorectal cancer progressing after therapy: the NIKOLO trial. BMC Cancer. 2018; 18:297
[31] SwissADME: a free web tool to evaluate pharmacokinetics, drug-likeness and medicinal chemistry friendliness of small molecules. Sci. Rep. (2017) 7:42717.
[32] Lipinski CA, Lombardo F, Dominy BW, Feeney PJ. Experimental and computational approaches to estimate solubility and permeability in drug discovery and development settings. Adv Drug Deliv Rev. 2001 Mar 1;46(1-3):3-26. doi: 10.1016/s0169-409x(00)00129-0. PMID: 11259830.
[33] Gross AS. Best practice in therapeutic drug monitoring. Br J Clin Pharmacol. 1998;46(2):95-99. doi:10.1046/j.1365-2125.1998.00770.x
[35] Sack U, Walther W, Scudiero D, et al. Novel effect of antihelminthic Niclosamide on S100A4-mediated metastatic progression in colon cancer. J Natl Cancer Inst. 2011;103(13):1018-1036. doi: 10.1 093/jnci/djr190
[36] Bairoch, A. The Cellosaurus, a Cell-Line Knowledge Resource. J. Biomol. Technol. 2018, 29, 25-38.
[37] Ilyas M, Tomlinson IP, Rowan A, Pignatelli M, Bodmer WF. Beta-catenin mutations in cell lines established from human colorectal cancers. Proc Natl Acad Sci U S A. 1997;94(19):10330-10334. doi:10.1073/pnas.94.19.10330
[38] Kinzler KW, Nilbert MC, Vogelstein B, et al. Identification of a gene located at chromosome 5q21 that is mutated in colorectal cancers. Science. 1991;251(4999):1366-1370. doi:10.1126/science.1848370
[39] Nishisho, I. Nakamura, Y. Miyoshi, Y. Mutations of chromosome 5q21 genes in FAP and colorectal cancer patients. Science. 1991; 253:665-669
[40] Korinek V, Barker N, Morin PJ, et al. Constitutive transcriptional activation by a beta-catenin-Tcf complex in APC-/- colon carcinoma. Science. 1997;275(5307):1784-1787. doi:10.1126/science.275.5307.1784
[41] Morin PJ, Sparks AB, Korinek V, et al. Activation of beta-catenin-Tcf signaling in colon cancer by mutations in beta-catenin or APC. Science. 1997;275(5307):1787-1790. doi:10.1126/science.275.5307.1787
[42] Luo F, Li J, Wu S, et al. Comparative profiling between primary colorectal carcinomas and metastases identifies heterogeneity on drug resistance. Oncotarget. 2016;7(39):63937-63949. doi:10.18632/oncotarget.11570
[43] Ciardiello F, Ciardiello D, Martini G, Napolitano S, Tabernero J, Cervantes A. Clinical management of metastatic colorectal cancer in the era of precision medicine. CA Cancer J Clin. 2022;72(4):372-401. doi:10.3322/caac.21728
[44] Hlubek F., Brabletz T., Budczies J., Pfeiffer S., Jung A., Kirchner T. Heterogeneous expression of Wnt/beta-catenin target genes within colorectal cancer. Int. J. cancer. 2007;121:1941-1948. doi: 10.1002/ijc.22916.
[45] Dahlmann M, Kobelt D, Walther W, Mudduluru G, Stein U. S100A4 in Cancer Metastasis: Wnt Signaling-Driven Interventions for Metastasis Restriction. Cancers (Basel). 2016;8(6):59. Published 2016 Jun 20. doi:10.3390/cancers8060059
[46] Kato J., Matsushime H., Hiebert S.W., Ewen M.E., Sherr C.J. Direct binding of cyclin D to the retinoblastoma gene product (pRb) and pRb phosphorylation by the cyclin D-dependent kinase CDK4. Genes Dev. 1993;7:331-342. doi: 10.1101/gad.7.3.331
[47] Lundberg A.S., Weinberg R.A. Functional Inactivation of the Retinoblastoma Protein Requires Sequential Modification by at Least Two Distinct Cyclin-cdk Complexes. Mol. Cell. Biol. 1998;18:753-761. doi: 10.1128/MCB.18.2.753
[48] Armelin HA, Armelin MC, Kelly K, Stewart T, Leder P, Cochran BH, et al. Functional role for c-myc in mitogenic response to platelet-derived growth factor. Nature. 1984;310:655-660. doi: 10.1038/310655a0.
[49] Askew D S, Ashmun R A, Simmons B C, Cleveland J L. Constitutive c-myc expression in an IL-3-dependent myeloid cell line suppresses cell cycle arrest and accelerates apoptosis. Oncogene. 1991;6:1915-1922.
[50] Dang CV. c-Myc target genes involved in cell growth, apoptosis, and metabolism. Mol Cell Biol. 1999;19(1):1-11. doi:10.1128/MCB.19.1.1
[51] Khramtsov, A. I., Khramtsova, G. F., Tretiakova, M., Huo, D., Olopade, O. I., and Goss, K. H. (2010). Wnt/β-catenin pathway activation is enriched in basal-like breast cancers and predicts poor outcome. Am. J. Pathol. 176, 2911-2920. doi: 10.2353/ajpath.2010.091125
[52] Arend RC, Londoño-Joshi Al, Straughn JM Jr, Buchsbaum DJ. The Wnt/β-catenin pathway in ovarian cancer: a review. Gynecol Oncol. 2013;131(3):772-779. doi:10.1016/j.ygyno.2013.09.034
[53] Stewart DJ. Wnt signaling pathway in non-small cell lung cancer. J Natl Cancer Inst. 2014;106(1):djt356. doi:10.1093/jnci/djt356
[54] Chiurillo MA. Role of the Wnt/β-catenin pathway in gastric cancer: An in-depth literature review. World J Exp Med. 2015;5(2):84-102. Published 2015 May 20. doi:10.5493/wjem.v5.i2.84
[55] Xue G, Romano E, Massi D, Mandalà M. Wnt/β-catenin signaling in melanoma: Preclinical rationale and novel therapeutic insights. Cancer Treat Rev. 2016;49:1-12. doi:10.1016/j.ctrv.2016.06.009
[56] Juneja, M., Kobelt, D., Walther, W., Voss, C., Smith, J., Specker, E., et al. Statin and rottlerin small-molecule inhibitors restrict colon cancer progression and metastasis via MACC1. PLoS Biol. 15, e2000784 (2017).
[57] Stein U, Arlt F, Smith J, et al., Intervening in β-Catenin Signaling by Sulindac Inhibits S100A4-Dependent Colon Cancer Metastasis Neoplasia. 2011 Feb;13(2):131-44
[58] Sack U, Walther W, Scudiero D et al., S100A4-induced cell motility and metastasis is restricted by the Wnt/β-catenin pathway inhibitor calcimycin in colon cancer cells Mol Biol Cell. 2011 Sep;22(18):3344-54
[59] Sack U, Walther W, Scudiero D, et al., Novel effect of antihelminthic Niclosamide on S100A4-mediated metastatic progression in colon cancer, J Natl Cancer Inst. 2011 Jul 6;103(13):1018-36).
[60] O'Reilly S, S100A4 a classical DAMP as a therapeutic target in fibrosis, Matrix Biol., 2024 Mar:127:1-7
[61] Cerezo L A, Remáková M, Tomčik M, et al., The metastasis-associated protein S100A4 promotes the inflammatory response of mononuclear cells via the TLR4 signalling pathway in rheumatoid arthritis, Rheumatology (Oxford), 2014 Aug; 53(8):1520-6
[62] Wang T, The function of S100A4 in pulmonary disease: A review, Medicine, 2022 Apr 07; 102(14):e33466.
[63] D'Ambrosi N, Milani M, Apolloni S, S100A4 in the Physiology and Pathology of the Central and Peripheral Nervous System, 2021 Apr 02; 10(4):798.
[64] Zhou Y, Zha Y, Yang Y, S100 proteins in cardiovascular diseases, Molecular Medicine, 2023, 29 (68)

## Claims

1. A compound according to **Formula 1** for use in the treatment of cancer 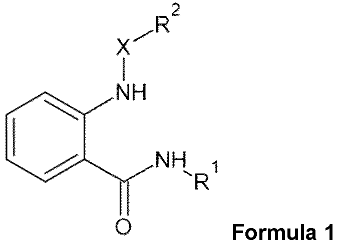 wherein
**X** is carbonyl -(C=O)- or sulfonyl -(SO₂)-,
**R¹** is a mono- or bicyclic heteroaryl group (preferably comprising N, S and/or O, more preferably an azole, thiazole or benzothiazol),
optionally substituted with one or more of C1 to C5 alkyl (preferably C1 to C3 alkyl), halogen (preferably F, Cl and/or Br), nitro, alkyl sulfonyl (preferably C1 to C3), alkoxy (preferably OCH₂CH₃), haloalkyl (preferably comprising F), hydroxyl, acyl, aryl (preferably phenyl, wherein said aryl is optionally substituted with C1 to C5 alkyl (preferably C1 to C3 alkyl) or halogen (preferably F, Cl and/or Br), and
R² is a mono- or bicyclic aryl group (preferably phenyl) or a bicyclic structure comprising an aryl group (preferably benzodioxanyl),
optionally substituted with one or more of C1 to C5 alkyl (preferably C1 to C3 alkyl), halogen (preferably F, Cl and/or Br), alkoxy (preferably OCH₂CH₃), haloalkyl (preferably comprising F, Cl and/or Br), hydroxyl, acyl.

2. The compound for use according to claim 1, according to **Formula 2,** 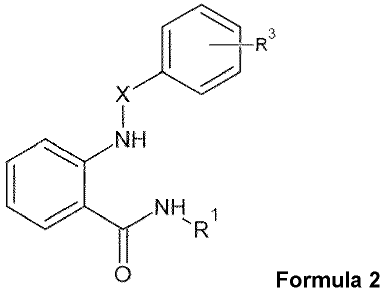 wherein
**X** and **R¹** are according to claim 1,
**R³** is one or more of C1 to C5 alkyl (preferably C1 to C3 alkyl), halogen (preferably F, Cl, Br and/or I), C1 to C5 alkoxy (preferably C1 to C3 alkoxy, wherein optionally two R³ groups form a ring structure fused with the adjacent phenyl), haloalkyl (preferably comprising F), hydroxyl, acyl.

3. The compound for use according to any one of the preceding claims, according to **Formula 3,** 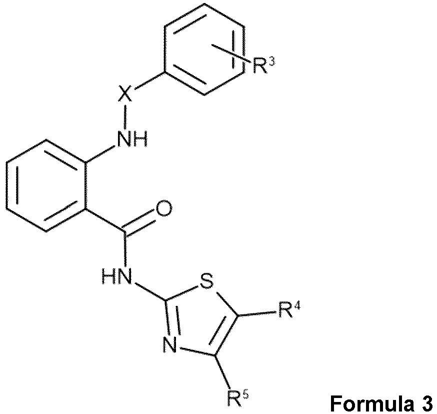 wherein
**X** is according to claim 1,
**R³** is according to claim 2,
**R⁴** is H, C1 to C5 alkyl (preferably C1 to C4 alkyl), optionally connected to **R⁵** forming a (preferably aromatic) ring structure fused with the adjacent thiazole (preferably forming a benzothiazole), aryl (preferably phenyl),
optionally substituted with one or more of C1 to C5 alkyl (preferably C1 to C3 alkyl), halogen (preferably F, Cl and/or Br), nitro, alkyl sulfonyl (preferably C1 to C3), alkoxy (preferably OCH₂CH₃), haloalkyl (preferably comprising F), hydroxyl, acyl, and
**R⁵** is H, C1 to C5 alkyl (preferably C1 to C4 alkyl), optionally connected to **R⁴** forming a (preferably aromatic) ring structure fused with the adjacent thiazole (preferably forming a benzothiazole), aryl (preferably phenyl),
optionally substituted with one or more of C1 to C5 alkyl (preferably C1 to C3 alkyl), halogen (preferably F, Cl and/or Br), nitro, alkyl sulfonyl (preferably C1 to C3), alkoxy (preferably OCH₂CH₃), haloalkyl (preferably comprising F), hydroxyl, acyl.

4. The compound for use according to any one of the preceding claims, according to **Formula 4,** 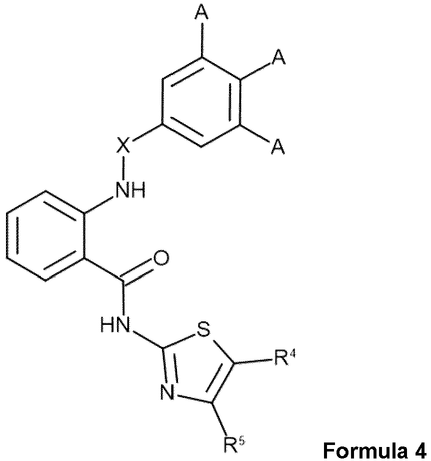 wherein
**X** is according to claim 1,
**R⁴** and **R⁵** are according to claim 3, and
A can be the same or different, H, C1 to C5 alkyl (preferably C1 to C3 alkyl), halogen (preferably F, Cl, Br and/or I), C1 to C5 alkoxy (preferably C1 to C3 alkoxy), wherein optionally two A groups form a ring structure with the adjacent phenyl, hydroxyl, acyl.

5. The compound for use according to any one of the preceding claims, according to **Formula 5** 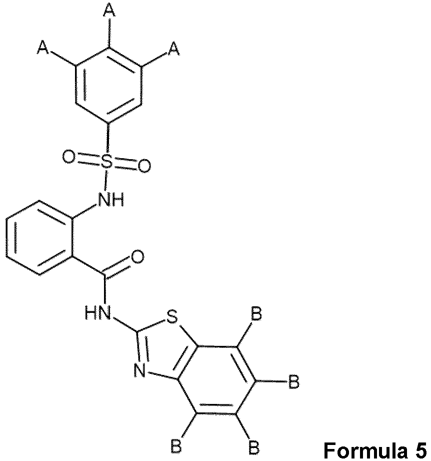 wherein
A is according to claim 4, and
B can be the same or different, H, C1 to C5 alkyl (preferably C1 to C3 alkyl), halogen (preferably F, Cl and/or Br), nitro, alkyl sulfonyl (preferably C1 to C3), alkoxy (preferably OCH₂CH₃), haloalkyl (preferably comprising F), hydroxyl, acyl.

6. The compound for use according to claim 1, according to **Formula 6** 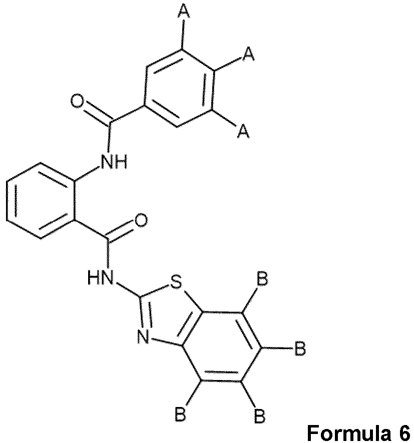 wherein
A is according to claim 4 and
B is according to claim 5

7. The compound for use according to any one of the preceding claims, wherein the compound is one of

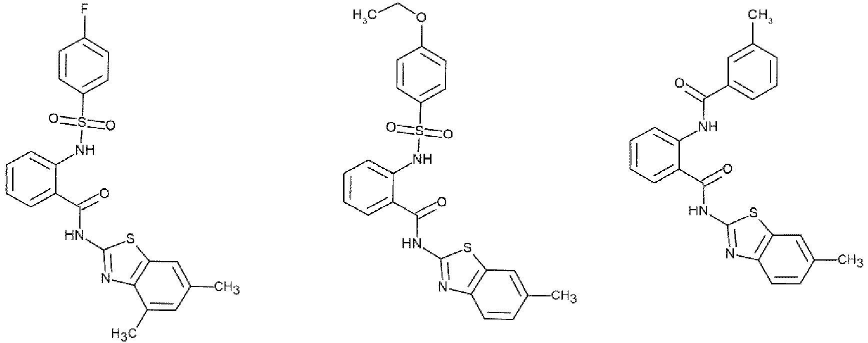

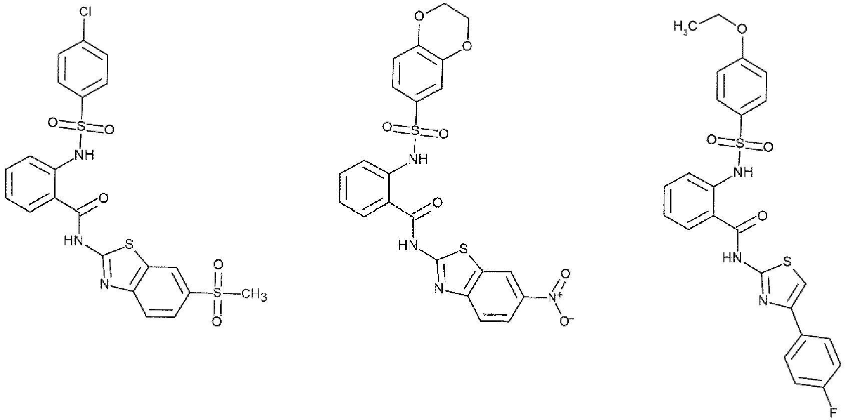

8. The compound for use according to any one of the preceding claims, wherein the compound inhibits transcription of S100A4.

9. The compound for use according to any one of the preceding claims, for use in the treatment of a cancer comprising transcription of S100A4 (preferably comprising S100A4 expression above levels in a non-cancerous cell).

10. The compound for use according to any one of the preceding claims, for use in the treatment of a cancer comprising an aberrant Wnt-signaling compared to a non-cancerous cell.

11. The compound for use according to any one of the preceding claims, for use in the treatment and/or prevention of cancer metastasis (preferably to inhibit metastatic cancer cell motility).

12. The compound for use according to any one of the preceding claims, for use in the treatment of a solid tumor, or a cancer selected from the group consisting of gastrointestinal, colorectal, gastric, esophageal, pancreatic, hepatocellular, biliary, lung, nasopharyngeal, renal, bladder, ovarian, brain, bone, head and neck, prostate, melanoma and breast cancer.

13. The compound for use according to any one of the preceding claims, wherein the cancer is selected from the group consisting of colorectal, gastric, esophageal, pancreatic, lung, bladder, ovarian and prostate cancer.

14. The compound for use according to any one of the preceding claims, for use in the treatment and/or prevention of colorectal cancer metastasis.

15. A pharmaceutical composition for use in the treatment of cancer, comprising one or more compounds according to any one of claims 1-7 or a pharmaceutically acceptable salt thereof, and a pharmaceutically acceptable carrier.
